# EUROPEAN PATENT APPLICATION

(11) **EP 4 226 945 A1**
(43) Date of publication of application: **16.08.2023**
(21) Application number: 21877781.1
(22) Date of filing: 04.10.2021
(51) Int. Cl.: A61K 45/00, A61K 31/496, A61K 31/506, A61K 31/517, A61K 39/395, A61K 47/68, A61P 35/00, A61P 43/00, C07K 16/18, C12N 15/13

(54) **MEDICINE FOR TREATING CANCER**

(30) Priority: 05.10.2020 JP 2020168522
(71) Applicant: Chiome Bioscience, Inc, Tokyo 151-0071 (JP)
(72) Inventor: NAKAMURA, Koji, Tokyo 151-0071 (JP); TAKAHASHI, Kota, Tokyo 151-0071 (JP); SAKAGUCHI, Izumi, Tokyo 151-0071 (JP)
(74) Representative: J A Kemp LLP
(86) International application number: PCT/JP2021/037494
(87) International publication number: WO 2022/075482

(57) **Abstract**

The present invention provides a therapeutic agent and a therapeutic method for cancer, etc., capable of exerting a more potent and sustained antitumor effect than existing FGFR4-selective tyrosine kinase inhibitors. The present invention is directed to a pharmaceutical combination for the treatment of cancer, characterized by comprising: a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and an antibody against human DLK-1, which has *in vivo* antitumor activity, or an antibody fragment derived from the antibody.

## Description

### Technical Field

The present invention relates to a medicine and a method for the treatment of cancer, etc.

### Background Art

Among various types of cancers, hepatic cancer is the second leading cause of cancer-related death in the world, and about 750,000 people die of hepatic cancer a year in the world. About 780,000 patients are newly diagnosed a year, and about 80% of them are found exclusively in the Asian region including Japan and China. Hepatocellular carcinoma accounts for 85% to 90% of all hepatic cancer cases. In Japan, it is reported that the number of patients with hepatocellular carcinoma is about 42,000 and the annual number of deaths is about 26,000. Unresectable hepatocellular carcinoma is a disease with limited therapeutic options, very poor prognosis and high unmet medical needs.

As a therapeutic agent for unresectable and progressive hepatocellular carcinoma, a multikinase inhibitor against multiple receptor tyrosine kinases has been used. In more detail, sorafenib or lenvatinib has been used as a first-line drug, and regorafenib or cabozantinib has been used as a second-line drug, but the effect obtained is limited.

In hepatic cancer cases, about 30% of patients are reported to overexpress fibroblast growth factor (FGF)-19 (Non-patent Document 1 and Non-patent Document 2), and it is reported that FGF-19 secreted in excess from hepatic cancer cells acts in an autocrine manner on its receptor, i.e., fibroblast growth factor receptor 4 (FGFR4) to cause the excessive activation of FGFR4 in hepatic cancer cells, thereby resulting in the enhanced proliferation of hepatic cancer cells and the further progression of hepatic cancer. Fibroblast growth factor receptor 4 (FGFR4), which is a tyrosine kinase receptor for FGF, is involved in diverse cellular processes, including the regulation of cell proliferation, differentiation, migration, metabolism, and bile acid biosynthesis, and high activation of FGFR4 is strongly associated with the amplification of its specific ligand FGF19 in many types of solid tumors and hematologic malignancies, where it acts as an oncogene driving the cancer development and progression. Moreover, in malignant cancers, FGFR4 expressed in cancer cells is activated in excess due to the overexpression of its ligand FGF19, which in turn enhances diverse cellular processes

(e.g., cell proliferation, differentiation, migration, metabolism) in cancer cells. For example, among patients with hepatic cancer, 30% of them are in this state. Currently, in the case of hepatocellular carcinoma, five types of inhibitors whose target molecule is FGFR4 are in clinical development stage (Non-patent Document 2). Among these five types of inhibitors, three types (FGF401, H3B-6527 and BLU-554) are small molecule TKIs (tyrosine kinase inhibitors), and their mechanism of action is to specifically bind to Cys552 in the ATP-binding region specific for FGFR4 and thereby inhibit the kinase activity. These FGFR4-selective small molecule tyrosine kinase inhibitors are now in clinical trials targeted for patients with progressive hepatocellular carcinoma overexpressing FGF-19 or expressing FGFR4. These FGFR4 inhibitors are all in the development stage of phase I or phase VII and therefore provide limited information about their efficacy, but the above inhibitors each have a limited therapeutic effect as monotherapy.

### Prior Art Documents

Non-patent Document 1: Diseases 2015, 3, 294-305
Non-patent Document 2: Cells. 2019, 8, 536

### Summary of the Invention

Under these circumstances, there has been a demand for the development of a therapeutic agent and a therapeutic method for cancer, etc., capable of exerting a more potent and sustained antitumor effect than existing FGFR4-selective tyrosine kinase inhibitors against various types of cancers, particularly cancers where FGFR4 is expressed and/or the tyrosine kinase activity of FGFR4 is observed, as exemplified by hepatocellular carcinoma.

The present invention has been made in consideration of the above situation and aims to provide a pharmaceutical combination for the treatment of cancer, etc., as shown below.
(1) A pharmaceutical combination for the treatment of cancer, comprising:
   a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
   an antibody against human DLK-1, which has *in vivo* antitumor activity, or an antibody fragment derived from the antibody.
(2) A pharmaceutical composition for the treatment of cancer, comprising an antibody against human DLK-1, which has *in vivo* antitumor activity, or an antibody fragment derived from the antibody,
   wherein the pharmaceutical composition is used in combination with a substance which inhibits or suppresses the tyrosine kinase activity of fibroblast growth factor receptor 4 (FGFR4), or a prodrug thereof, or a pharmacologically acceptable salt thereof, or a hydrate or solvate thereof.
(3) A pharmaceutical composition for the treatment of cancer, comprising a substance which inhibits or suppresses the tyrosine kinase activity of fibroblast growth factor receptor 4 (FGFR4), or a prodrug thereof, or a pharmacologically acceptable salt thereof, or a hydrate or solvate thereof,
   wherein the pharmaceutical composition is used in combination with an antibody against human DLK-1, which has *in vivo* antitumor activity, or an antibody fragment derived from the antibody.
(4) A pharmaceutical composition for the treatment of cancer, comprising an antibody against human DLK-1, which has *in vivo* antitumor activity, or an antibody fragment derived from the antibody,
   wherein the treatment comprises administering a substance which inhibits or suppresses the tyrosine kinase activity of fibroblast growth factor receptor 4 (FGFR4), or a prodrug thereof, or a pharmacologically acceptable salt thereof, or a hydrate or solvate thereof.
(5) A pharmaceutical composition for the treatment of cancer, comprising a substance which inhibits or suppresses the tyrosine kinase activity of fibroblast growth factor receptor 4 (FGFR4), or a prodrug thereof, or a pharmacologically acceptable salt thereof, or a hydrate or solvate thereof,
   wherein the treatment comprises administering an antibody against human DLK-1, which has *in vivo* antitumor activity, or an antibody fragment derived from the antibody.
(6) The pharmaceutical combination according to (1) above, or the pharmaceutical composition according to any one of (2) to (5) above, wherein the substance which inhibits or suppresses the tyrosine kinase activity of fibroblast growth factor receptor 4 (FGFR4) is H3B-6527, FGF-401 (roblitinib) or BLU-554 (fisogatinib).
(7) The pharmaceutical combination according to (1) above, or the pharmaceutical composition according to any one of (2) to (5) above, wherein the cancer and/or the tumor is a cancer where FGFR4 is expressed and/or the tyrosine kinase activity of FGFR4 is observed.
(8) The pharmaceutical combination according to (1) above, or the pharmaceutical composition according to any one of (2) to (5) above, wherein the cancer and/or the tumor is at least one selected from the group consisting of hepatocellular carcinoma, lung cancer, uterine body cancer, cholangiocarcinoma, intrahepatic bile duct cancer, esophageal cancer, nasopharyngeal cancer, ovarian cancer, breast cancer, renal cell carcinoma, pancreatic cancer, colon cancer, and glioblastoma, as well as sarcomas including rhabdomyosarcoma.
(9) The pharmaceutical combination according to (1) above, or the pharmaceutical composition according to any one of (2) to (5) above, wherein the antibody is a chimeric antibody or a humanized antibody.
(10) The pharmaceutical combination according to (1) above, or the pharmaceutical composition according to any one of (2) to (5) above, wherein the antibody is at least one selected from the group consisting of:
   (a) an antibody in which the amino acid sequences of H chain V region CDRs 1 to 3 are the amino acid sequences shown in SEQ ID NOs: 3 to 5, respectively, and the amino acid sequences of L chain V region CDRs 1 to 3 are the amino acid sequences shown in SEQ ID NOs: 6 to 8, respectively;
   (b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
   (c) an antibody in which the amino acid sequence of the H chain V region consists of the amino acid sequence shown in SEQ ID NO: 16, and the amino acid sequence of the L chain V region consists of the amino acid sequence shown in SEQ ID NO: 18;
   (d) an antibody in which the amino acid sequence of the H chain V region consists of the amino acid sequence shown in SEQ ID NO: 20, and the amino acid sequence of the L chain V region consists of the amino acid sequence shown in SEQ ID NO: 22;
   (e) an antibody in which the amino acid sequence of the H chain V region consists of the amino acid sequence shown in SEQ ID NO: 24 or 26, and the amino acid sequence of the L chain V region consists of the amino acid sequence shown in SEQ ID NO: 28;
   (f) an antibody in which the amino acid sequence of the H chain V region consists of the amino acid sequence shown in SEQ ID NO: 30, 32, 34 or 36, and the amino acid sequence of the L chain V region consists of the amino acid sequence shown in SEQ ID NO: 46;
   (g) an antibody in which the amino acid sequence of the H chain V region consists of the amino acid sequence shown in SEQ ID NO: 38, 40, 42 or 44, and the amino acid sequence of the L chain V region consists of the amino acid sequence shown in SEQ ID NO: 46;
   (h) an antibody produced by the hybridoma of Accession No. FERM BP-10899;
   (i) an antibody produced by the hybridoma of Accession No. FERM BP-10707;
   (j) an antibody produced by the hybridoma of Accession No. FERM BP-10900; and
   (k) an antibody produced by the hybridoma of Accession No. FERM BP-11337.
(11) The pharmaceutical combination according to (1) above, or the pharmaceutical composition according to any one of (2) to (5) above, wherein the antibody or antibody fragment is in the form of a conjugate with a compound having antitumor activity and/or cell killing activity.
(12) The pharmaceutical combination according to (1) above, or the pharmaceutical composition according to any one of (2) to (5) above, which allows suppression of cancer cell proliferation or allows tumor reduction or disappearance even after completing the administration of the pharmaceutical combination or pharmaceutical composition.
(13) The use of:
   a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
   an antibody against human DLK-1, which has *in vivo* antitumor activity, or an antibody fragment derived from the antibody
   for the manufacture of a therapeutic agent for cancer.
(14) A therapeutic method for cancer, characterized by administering a subject with:
   a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
   an antibody against human DLK-1, which has *in vivo* antitumor activity, or an antibody fragment derived from the antibody.
(15) A kit for the treatment of cancer, comprising:
   a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
   an antibody against human DLK-1, which has *in vivo* antitumor activity, or an antibody fragment derived from the antibody.
(16) The use according to (13) above, the method according to (14) above, or the kit according to (15) above, wherein the cancer and/or the tumor is a cancer where FGFR4 is expressed and/or the tyrosine kinase activity of FGFR4 is observed.

### Effects of the Invention

The present invention enables the provision of a therapeutic agent and a therapeutic method, etc., capable of exerting a more potent and sustained antitumor effect than existing tyrosine kinase inhibitors in the treatment of cancer, particularly in the treatment of cancers where FGFR4 is expressed and/or the tyrosine kinase activity of FGFR4 is observed. The therapeutic agent and therapeutic method, etc., of the present invention are very useful, for example, in term of being capable of exerting an effect on patients who could not have been expected to experience any therapeutic effect.

### Brief Description of the Drawings

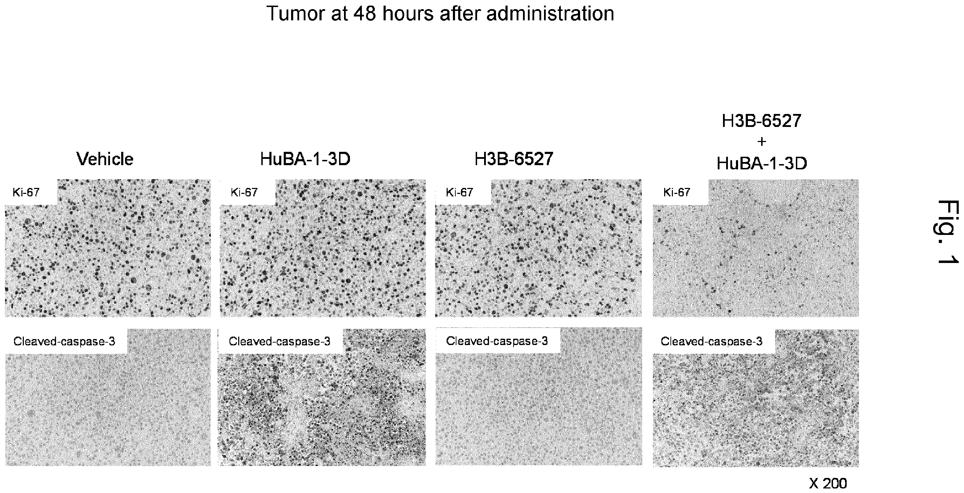

Figure 1 shows photographs of immunostaining for cell proliferation (Ki-67) and apoptosis (cleaved caspase-3) at 48 hours after administration in the Hep3B xenograft model in the Vehicle group, the group receiving HuBA-1-3D (1 mg/kg), the group receiving H3B-6527 (50 mg/kg) and the group receiving HuBA-1-3D (1 mg/kg) + H3B-6527 (50 mg/kg). Spots stained dark brown indicate proliferating (Ki-67-positive) or apoptotic (cleaved caspase-3-positive) cells.

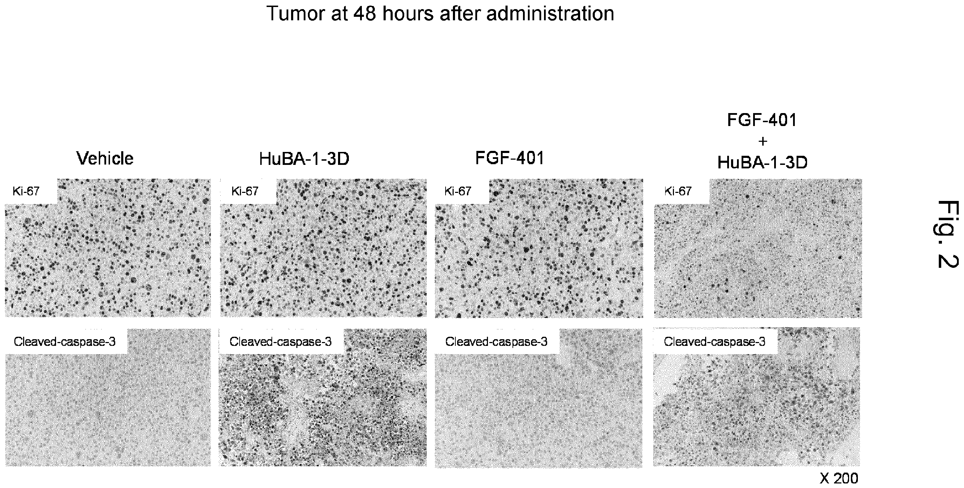

Figure 2 shows photographs of immunostaining for cell proliferation (Ki-67) and apoptosis (cleaved caspase-3) at 48 hours after administration in the Hep3B xenograft model in the Vehicle group, the group receiving HuBA-1-3D (1 mg/kg), the group receiving FGF-401 (10 mg/kg) and the group receiving HuBA-1-3D (1 mg/kg) + FGF-401 (10 mg/kg). Spots stained dark brown indicate proliferating (Ki-67-positive) or apoptotic (cleaved caspase-3-positive) cells.

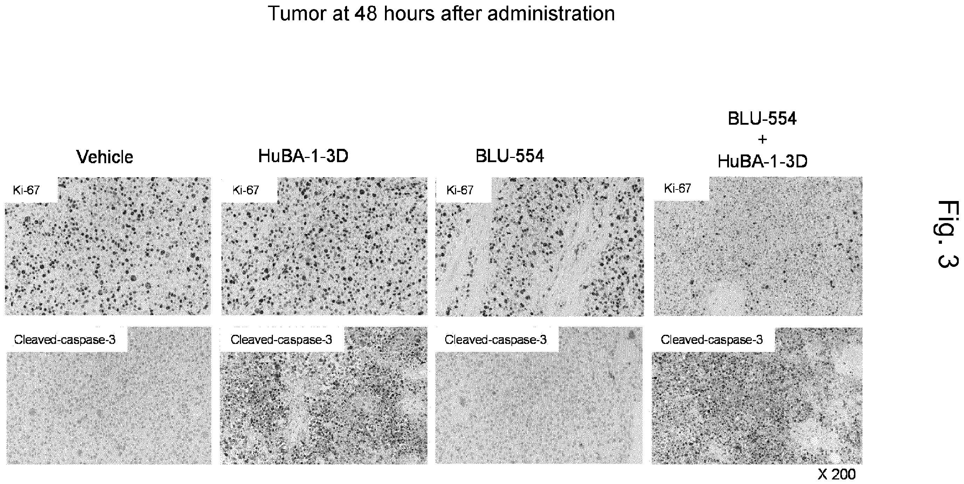

Figure 3 shows photographs of immunostaining for cell proliferation (Ki-67) and apoptosis (cleaved caspase-3) at 48 hours after administration in the Hep3B xenograft model in the Vehicle group, the group receiving HuBA-1-3D (1 mg/kg), the group receiving BLU-554 (30 mg/kg) and the group receiving HuBA-1-3D (1 mg/kg) + BLU-554 (30 mg/kg). Spots stained dark brown indicate proliferating (Ki-67-positive) or apoptotic (cleaved caspase-3-positive) cells.

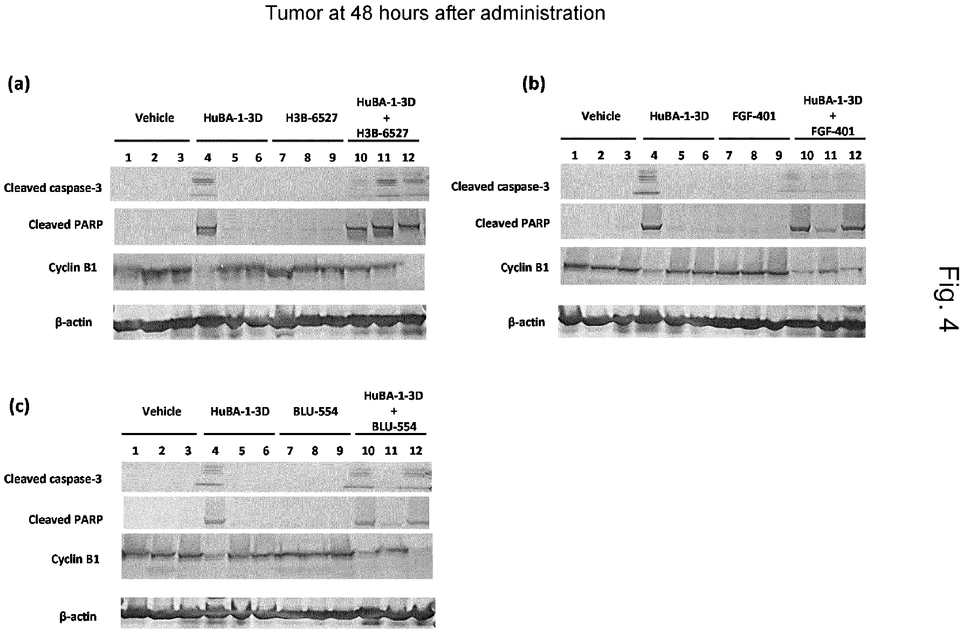

Figure 4 shows the expression of apoptosis markers (cleaved caspase-3 and cleaved PARP) and cell proliferation marker (cyclin B1) in tumor at 48 hours after administration of Vehicle, HuBA-1-3D (1 mg/kg), FGFR4-selective inhibitors (H3B-6527 (50 mg/kg), FGF-401 (10 mg/kg) and BLU-554 (30 mg/kg)), and HuBA-1-3D (1 mg/kg) in combination with each FGFR4 inhibitor in the Hep3B xenograft model, as analyzed by Western blotting. (a) H3B-6527, (b) FGF-401, (c) BLU-554. N = 3.

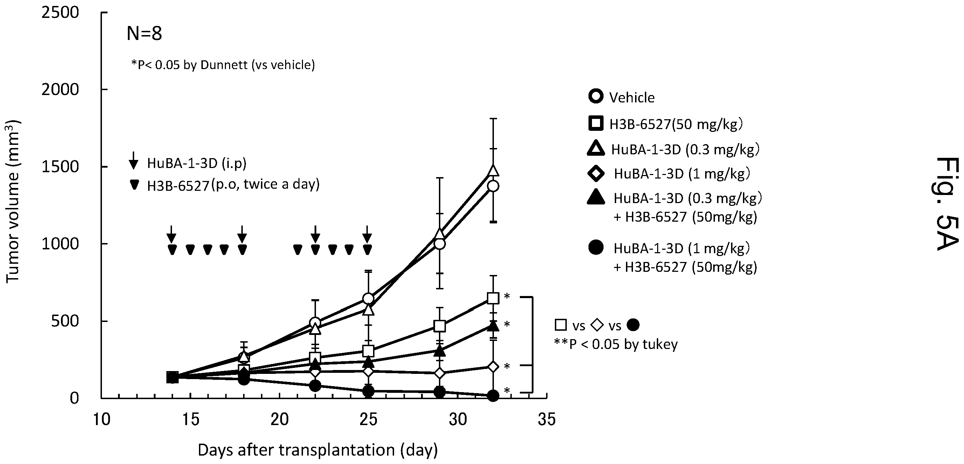

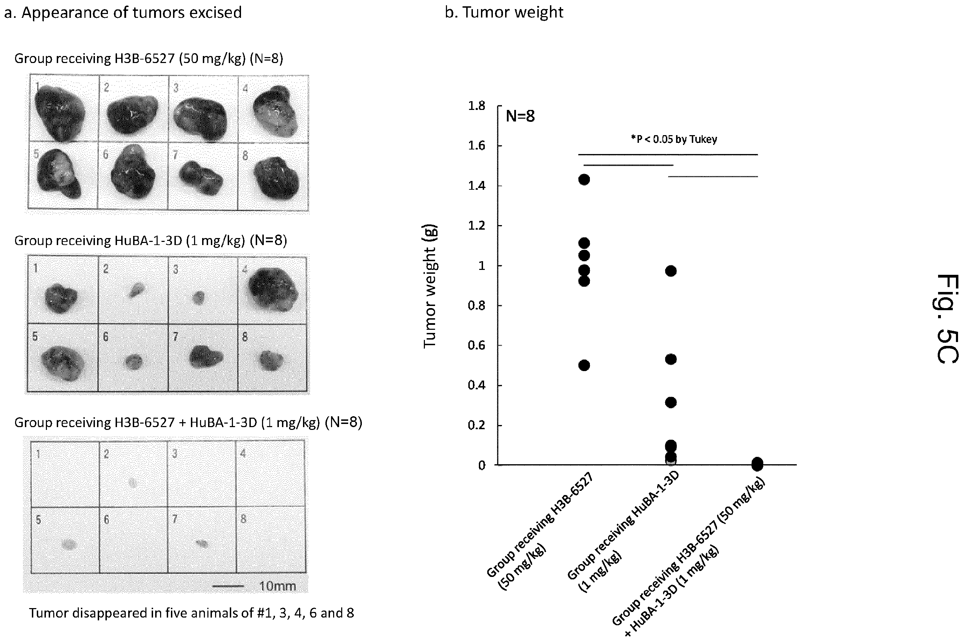

Figure 5A shows the antitumor effect enhanced upon combined administration of HuBA-1-3D antibody and FGFR4-selective inhibitor (H3B-6527).
   In more detail, Figure 5A is a graph showing tumor growth until Day 32 after transplantation (i.e., until 7 days after the final administration) in the Hep3B xenograft model in the Vehicle group (open circles) and upon administration of HuBA-1-3D alone (open triangles, open diamonds), administration of H3B-6527 alone (open squares), and combined administration of HuBA-1-3D and H3B-6527 (solid triangles, solid circles).

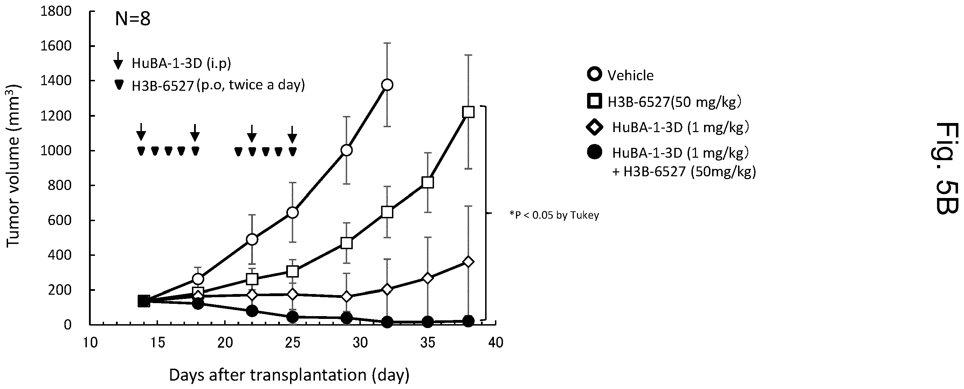

Figure 5B shows the antitumor effect enhanced upon combined administration of HuBA-1-3D antibody and FGFR4-selective inhibitor (H3B-6527).
   In more detail, Figure 5B is a graph showing the time course of tumor volume observed until Day 38 (i.e., until 13 days after the final day of administration (Day 25)) in the group receiving H3B-6527 (50 mg/kg) (open squares), the group receiving HuBA-1-3D (1 mg/kg) (open diamonds) and the group receiving H3B-6527 (50 mg/kg) + HuBA-1-3D (1 mg/kg) (solid circles).
Figure 5C shows the antitumor effect enhanced upon combined administration of HuBA-1-3D antibody and FGFR4-selective inhibitor (H3B-6527).
   In more detail, Figure 5C shows (a) photographs of tumor appearance and (b) a graph of tumor weight in tumors excised on Day 38 (i.e., at 13 days after the final day of administration (Day 25)) from the group receiving H3B-6527 (50 mg/kg), the group receiving HuBA-1-3D (1 mg/kg) and the group receiving H3B-6527 (50 mg/kg) + HuBA-1-3D (1 mg/kg).

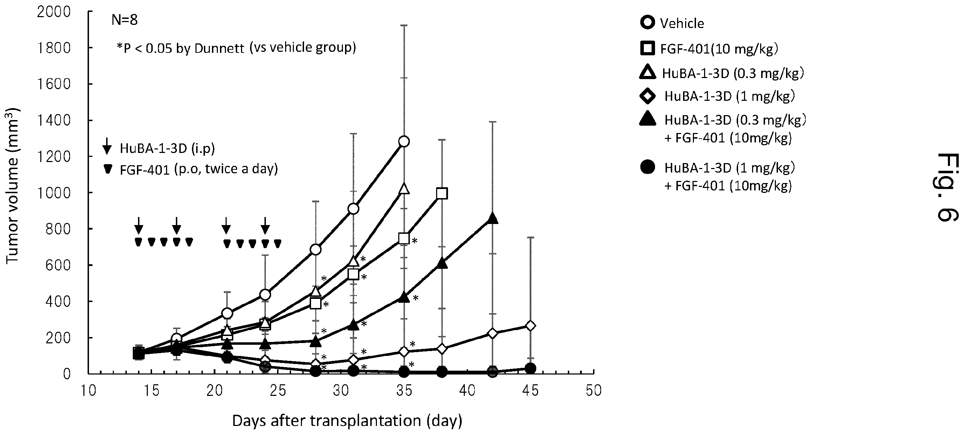

Figure 6 shows the antitumor effect enhanced upon combined administration of HuBA-1-3D antibody and FGFR4-selective inhibitor (FGF-401).
   In more detail, Figure 6 is a graph showing tumor growth in the Hep3B xenograft model in the Vehicle group (open circles), the group receiving HuBA-1-3D alone (open triangles, open diamonds), the group receiving FGF-401 alone (open squares) and the group receiving HuBA-1-3D and FGF-401 (solid triangles, solid circles). An animal whose tumor volume reached 1,500 mm³ was sacrificed. The graph shows data until the point of time when all the animals (N = 8 for each group) survived in each tested group.

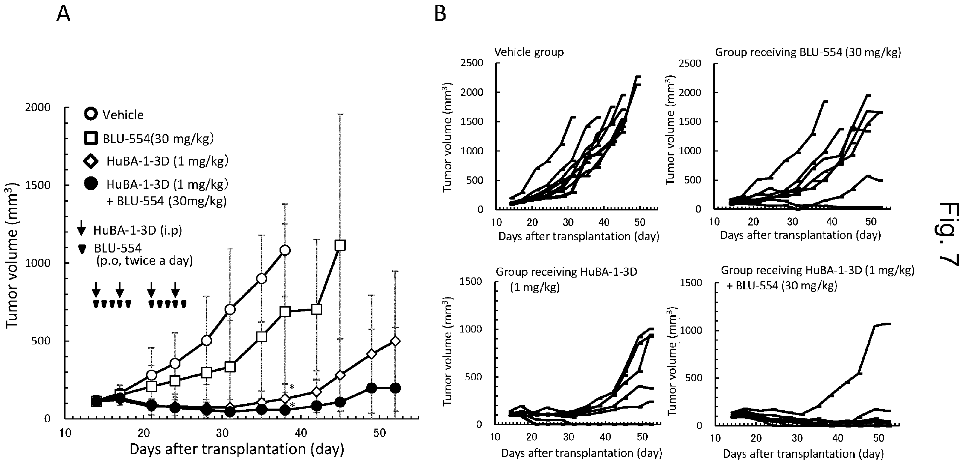

Figure 7 shows the antitumor effect enhanced upon combined administration of HuBA-1-3D antibody and FGFR4-selective inhibitor (BLU-554).
   In more detail, (A) a graph showing the mean tumor growth in the Hep3B xenograft model in the Vehicle group (open circles), the group receiving HuBA-1-3D alone (open diamonds), the group receiving BLU-554 alone (open squares) and the group receiving HuBA-1-3D and BLU-554 (solid circles). An animal whose tumor volume reached 1,500 mm³ was sacrificed. The graph shows data until the point of time when at least 7 animals survived in each tested group (N = 8 for each group). *P<0.05 (vs the Vehicle group, Dunnett). (B) Graphs showing tumor growth per animal in the Vehicle group, the group receiving HuBA-1-3D alone, the group receiving BLU-554 alone and the group receiving HuBA-1-3D and BLU-554.

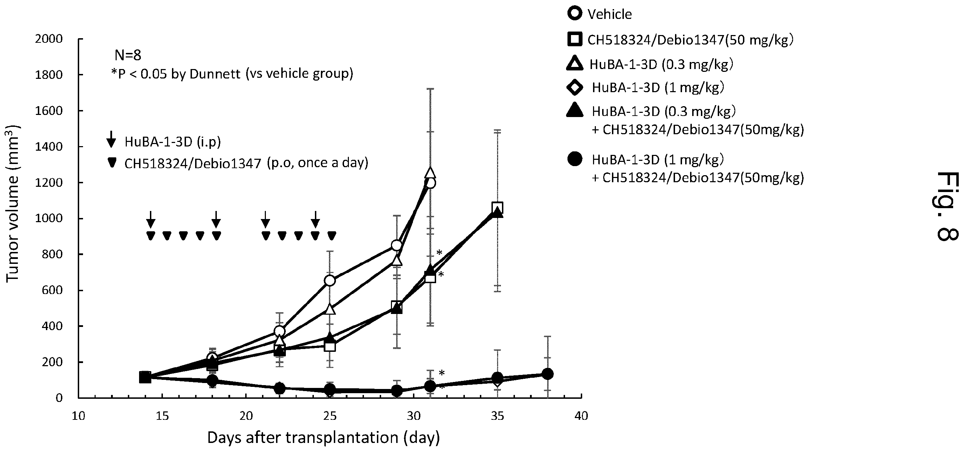

Figure 8 shows the antitumor effect enhanced upon combined administration with an inhibitor against FGFR1/FGFR2/FGFR3 (CH518324/Debio1347).
   In more detail, Figure 8 is a graph showing tumor growth in the Hep3B xenograft model in the Vehicle group (open circles), the group receiving HuBA-1-3D alone (open triangles, open diamonds), the group receiving CH518324/Debio1347 alone (open squares) and the group receiving HuBA-1-3D and CH518324/Debio1347 (solid triangles, solid circles). An animal whose tumor volume reached 1,500 mm³ was sacrificed. The graph shows data until the point of time when all the animals (N = 8 for each group) survived in each tested group.

### Description of Embodiments

The present invention will be further described in more detail below. The scope of the present invention is not limited by the following descriptions, and any embodiments other than those illustrated below may also be carried out with appropriate modifications without departing from the spirit of the present invention. It should be noted that this specification incorporates the specification of Japanese Patent Application No. 2020-168522 (filed on October 5, 2020) in its entirety, based on which the present application claims priority. Moreover, all publications cited herein, including prior art documents, patent gazettes and other patent documents, are incorporated herein by reference.

### 1. Summary of the present invention

As described above, currently, in the case of hepatocellular carcinoma, which is an example of cancers where FGFR4 is expressed and/or the tyrosine kinase activity of FGFR4 is observed, five types of inhibitors whose target molecule is FGFR4 are in clinical development stage, of which three types (FGF-401, H3B-6527 and BLU-554) are small molecule TKIs (tyrosine kinase inhibitors), and their mechanism of action is to specifically bind to Cys552 in the ATP-binding region specific for FGFR4 and thereby inhibit the kinase activity. These small molecule tyrosine kinase inhibitors are now in clinical trials targeted for patients with progressive hepatocellular carcinoma overexpressing FGF-19 or expressing FGFR4, and they are all in the development stage of phase I or phase VII and therefore provide limited information about their efficacy, but the above inhibitors each have a limited therapeutic effect as monotherapy.

To develop a therapeutic agent capable of exerting a more potent and sustained antitumor effect than the above existing tyrosine kinase inhibitors, the inventors of the present invention have made experiments and studies in a xenograft therapeutic model based on the human hepatocellular carcinoma-derived cell line Hep3B. As a result, the inventors of the present invention have elucidated that when such an FGFR4-selective tyrosine kinase inhibitor as mentioned above is administered in combination with an antibody against human DLK-1 (delta-like 1 homolog (Drosophila); hereinafter also referred to as "hDLK-1"), this combined administration exerts a more sustained and significantly potent tumor reduction effect than when they are administered alone. It should be noted that when a small molecule inhibitor against fibroblast growth factor receptors 1, 2 and 3 (FGFRs 1, 2 and 3) was administered in combination with an anti-hDLK-1 antibody, no enhancement in their antitumor effect was observed.

hDLK-1 is a single transmembrane type I membrane protein composed of 383 amino acid residues and is known to be expressed in adult cancers such as hepatocellular carcinoma, small cell lung cancer, pancreatic cancer, breast cancer, etc., and childhood cancers such as neuroblastoma, hepatoblastoma, rhabdomyosarcoma, virus tumor, etc. On the other hand, in normal tissues and organs, DLK-1 expression is limited to adrenal glands and pituitary glands, etc., and DLK-1 is not expressed in most organs, so that DLK-1 is suitable as a target molecule for cancer treatment. Until now, there have been developed anti-hDLK-1 monoclonal antibodies exerting tumor cell proliferation inhibition and tumor cell death induction effects including a high tumor reduction effect, and the antitumor activity of these antibodies when administered alone has been confirmed in xenograft therapeutic models using a plurality of human cancer cell lines including human hepatic cancer cell lines.

According to the combination medicament and combination therapy using an FGFR4-selective tyrosine kinase inhibitor and an anti-hDLK-1 antibody found by the inventors of the present invention, a sufficient therapeutic effect can be expected in cancer patients who could not have been expected to experience a sufficient therapeutic effect (or who have been nonresponders) when administered with the above existing tyrosine kinase inhibitors alone. The present invention was completed in this way.

### 2. Pharmaceutical combination for the treatment of cancer

As described above, the pharmaceutical combination for the treatment of cancer according to the present invention (hereinafter also referred to as "the pharmaceutical combination of the present invention") is characterized by comprising the following as active ingredients:
a substance which inhibits or suppresses the tyrosine kinase activity of fibroblast growth factor receptor 4 (FGFR4) (hereinafter also referred to as an "FGFR4 inhibitor"), or a prodrug thereof, or a pharmacologically acceptable salt thereof, or a hydrate or solvate thereof (hereinafter also referred to as an "FGFR4 inhibitor or other form thereof'); and
an anti-hDLK-1 antibody having *in vivo* antitumor activity, or an antibody fragment derived from the antibody (hereinafter also referred to as an "anti-hDLK-1 antibody or a fragment thereof').

In the present invention, the cancers to be treated are not limited in any way, but preferably exemplified by cancers where FGFR4 is expressed and/or the tyrosine kinase activity of FGFR4 is observed. More specifically, preferred examples include hepatocellular carcinoma, lung cancer, uterine body cancer, cholangiocarcinoma, intrahepatic bile duct cancer, esophageal cancer, nasopharyngeal cancer, and ovarian cancer, breast cancer, renal cell carcinoma, pancreatic cancer, colon cancer, and glioblastoma, as well as sarcomas including rhabdomyosarcoma, etc. Moreover, there is also no limitation on the "tumor" in the antitumor activity of an anti-hDLK-1 antibody, but the same explanation as given to the above cancers to be treated can also be applied to the tumor.

It should be noted that the present invention also includes: (i) a therapeutic method for cancer, which comprises using an FGFR4 inhibitor or other form thereof and an anti-hDLK-1 antibody or a fragment thereof, more specifically, for example, administering effective amounts of an FGFR4 inhibitor or other form thereof and an anti-hDLK-1 antibody or a fragment thereof to a subject (i.e., a cancer patient or a patient with a risk (onset risk) of cancer, or a non-human mammal in such a state); (ii) the use of an FGFR4 inhibitor or other form thereof and an anti-hDLK-1 antibody or a fragment thereof for the manufacture of a therapeutic agent for cancer; (iii) the use of an FGFR4 inhibitor or other form thereof and an anti-hDLK-1 antibody or a fragment thereof for the treatment of cancer; and (iv) an FGFR4 inhibitor or other form thereof and an anti-hDLK-1 antibody or a fragment thereof for use in the treatment of cancer.

In the present invention, the treatment of cancer also includes, for example, suppression of cancer progression, improvement of prognosis and/or prevention of recurrence, etc.

### (1) FGFR4 inhibitor or other form thereof

An FGFR4 inhibitor or other form thereof for use as an active ingredient in the pharmaceutical combination of the present invention is not limited in any way as long as it is a substance having the function of being capable of inhibiting or suppressing the tyrosine kinase activity of FGFR4. Such an FGFR4 inhibitor is preferably exemplified by FGFR4-selective tyrosine kinase inhibitors such as H3B-6527, FGF-401 (roblitinib) and BLU-554 (fisogatinib), etc. Such an FGFR4 inhibitor or other form thereof may be synthesized, extracted and purified independently for this purpose, or may be any commercially available product. In the case of being synthesized, extracted and purified independently, reference may be made to, for example, the descriptions in WO 2015/057938 (compound 108, pages 42-45) for H3B-6527, the descriptions in WO 2015/059668 (Example 83) for FGF-401, and the descriptions in WO 2015/061572 A1 for BLU-554.

H3B-6527 has an official name (IUPAC name) of N-{2-[(6-{[(2,6-dichloro-3, 5-dimethoxyphenyl)carbamoyl] (methyl)amino}-4-pyrimidinyl)amino]-5-(4-ethyl-1-piperazinyl)phenyl}acrylamide, and is represented by the structural formula shown below.

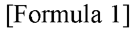

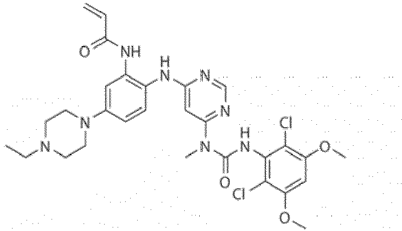

Likewise, FGF-401 (roblitinib) has an official name (IUPAC name) of N-[5-cyano-4-[(2-methoxyethyl)amino]-2-pyridinyl]-7-formyl-3,4-dihydro-6-[(4-methyl-2-oxo-1-piperazinyl)methyl]-1,8-naphthyridine-1(2H)-carboxamide, and is represented by the structural formula shown below.

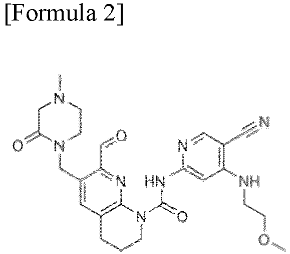

Likewise, BLU-554 (fisogatinib) has an official name (IUPAC name) of N-[(3S,4S)-3-[[6-(2,6-dichloro-3,5-dimethoxyphenyl)quinazolin-2-yl]amino]oxan-4-yl]prop-2-enamide, and is represented by the structural formula shown below.

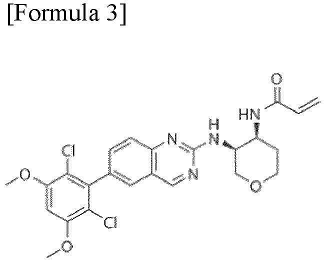

As an active ingredient in the pharmaceutical combination of the present invention, a derivative of the FGFR4 inhibitor may be used in combination with the FGFR4 inhibitor or in place of the FGFR4 inhibitor. Such a derivative is not limited in any way as long as it is considered to be a derivative of the FGFR4 inhibitor on the basis of common knowledge shared among those skilled in the art, e.g., in terms of having a chemical structure derived from the FGFR4 inhibitor, but preferred is a derivative having inhibitory or suppressive activity against the tyrosine kinase activity of FGFR4 at the same level as the FGFR4 inhibitor. In the present invention, when simply referred to as an FGFR4 inhibitor, it is intended to mean that a derivative of the FGFR4 inhibitor is also encompassed.

Examples of FGFR4 inhibitors for use in the present invention include not only those which undergo *in vivo* metabolism such as oxidation, reduction, hydrolysis or conjugation, but also compounds which produce FGFR4 inhibitors upon *in vivo* metabolism such as oxidation, reduction or hydrolysis (i.e., so-called prodrugs). In the present invention, such a prodrug refers to a compound prepared from its parent compound by modification with a pharmacologically acceptable group which is commonly used in prodrugs, as exemplified by a compound which is provided with properties such as improved stability and sustainability, and can be expected to exert the intended effect when converted into the parent compound in the intestinal tract or elsewhere. For example, a prodrug of the FGFR4 inhibitor can be prepared in a standard manner by using a prodrug-forming reagent such as a corresponding halide to introduce a prodrug-constituting group(s) as appropriate in a standard manner into any one or more groups selected from among the groups in this compound, which can be used for prodrug formation (e.g., a hydroxyl group, an amino group, other groups), optionally followed by isolation and purification. As intended here, the above prodrug-constituting groups preferably include, but are not limited to, lower alkyl-CO-, lower alkyl-O-lower alkylene-CO-, lower alkyl-OCO-lower alkylene-CO-, lower alkyl-OCO-, and lower alkyl-O-lower alkylene-OCO-, etc.

As an active ingredient in the pharmaceutical combination of the present invention, a pharmacologically acceptable salt of the FGFR4 inhibitor or a prodrug thereof may be used in combination with the FGFR4 inhibitor or a prodrug thereof or in place of the FGFR4 inhibitor or a prodrug thereof.

Such a pharmacologically acceptable salt is not limited in any way, but preferred examples include organic sulfonic acid salts (e.g., methanesulfonate (which is also referred to as mesylate), trifluoromethanesulfonate, ethanesulfonate, benzenesulfonate, toluenesulfonate, and camphorsulfonate), organic carboxylic acid salts (e.g., acetate, trifluoroacetate, maleate, tartrate, fumarate, and citrate), amino acid salts (e.g., aspartate, and glutamate), quaternary amine salts, alkali metal salts (e.g., sodium salt, and potassium salt), inorganic acid salts (e.g., sulfate, nitrate, perchlorate, phosphate, carbonate, and bicarbonate), halogenated hydroacid salts (e.g., hydrochloride, hydrobromide, and hydroiodide), alkaline earth metal salts (e.g., magnesium salt, and calcium salt) and so on.

The FGFR4 inhibitor for use in the present invention encompasses all isomers possible in terms of the compound's structure (e.g., geometrical isomers, optical isomers based on asymmetric carbons, rotational isomers, stereoisomers, and tautomers) and mixtures of two or more of these isomers, and is not limited to the descriptions about the structural formula shown for convenience' sake. Moreover, the FGFR4 inhibitor may be in S-configuration, R-configuration or RS-configuration, and is not limited in any way. Further, the FGFR4 inhibitor may be present in the form of a hydrate or solvate, depending on its type. In the present invention, such a hydrate or solvate also falls within the FGFR4 inhibitor, and may be used as an active ingredient in the pharmaceutical combination of the present invention. Such a solvate is not limited in any way, but is exemplified by a solvate with ethanol, etc.

In the pharmaceutical combination of the present invention, the content of the FGFR4 inhibitor or other form thereof as an active ingredient is not limited in any way and may be determined as appropriate, but may be set to be within the range of 0.01% to 99.99% by weight, relative to the total weight of the pharmaceutical combination, and preferably set to be within the range of 0.01% to 30% by weight, more preferably 0.05% to 20% by weight, even more preferably 0.1% to 10% by weight, relative to the total weight of the pharmaceutical combination. The content of the active ingredient within the above range is sufficient for the pharmaceutical combination of the present invention to exert a therapeutic effect on cancer.

In addition to the FGFR4 inhibitor or other form thereof, the pharmaceutical combination of the present invention may further comprise any other multikinase inhibitors, etc., as long as the effect of the present invention is not significantly impaired.

### (2) Anti-hDLK-1 antibody or a fragment thereof

An anti-hDLK-1 antibody (i.e., an antibody against human DLK-1, which has *in vivo* antitumor activity) for use as an active ingredient in the pharmaceutical combination of the present invention may be prepared on the basis of the following explanation.

### (i) Antigen preparation

Information on the amino acid sequence (SEQ ID NO: 2) of hDLK-1 has been made public, e.g., on the website of NCBI (GenBank) (http://www.ncbi.nlm.nih.gov/) under "Accession number: NP_003827." It should be noted that information on the nucleotide sequence (SEQ ID NO: 1) encoding the amino acid sequence of hDLK-1 has been made public on the same website under "Accession number: NM 003836."

As an antigen, it is possible to use a polypeptide or peptide (hereinafter also simply referred to as a peptide) comprising at least a part (the whole or a part) of the amino acid sequence of hDLK-1, preferably a peptide comprising at least a part (the whole or a part) of the amino acid sequence of the extracellular region (FA-1) of hDLK-1. The extracellular region of hDLK-1 refers to a region comprising six EGF-like motifs (EGF-1 to EGF-6) as described above, i.e., a region comprising amino acids at positions 24 to 244 in the amino acid sequence shown in SEQ ID NO: 2, preferably a region consisting of amino acids at positions "24" to "248 to 285" in the amino acid sequence shown in SEQ ID NO: 2 (approximately 225 to 262 amino acid residues).

As to the peptide for use as an antigen, the above "at least a part of the amino acid sequence" has no limitation on its length, and preferred is, for example, a region comprising one or two or more of the six EGF-like motifs. More preferred are, for example, a region comprising EGF-1 and EGF-2 (i.e., a region consisting of amino acids at positions 24 to 91 in the amino acid sequence shown in SEQ ID NO: 2), a region comprising EGF-3 and EGF-4 (i.e., a region consisting of amino acids at positions 92 to 167 in the amino acid sequence shown in SEQ ID NO: 2), and a region comprising EGF-4, EGF-5 and EGF-6 (i.e., a region consisting of amino acids at positions 131 to 244 in the amino acid sequence shown in SEQ ID NO: 2).

The peptide for use as an antigen may be prepared either by chemical synthesis or by synthesis through genetic engineering procedures using *E. coli* or the like, and techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., PSSM-8, Shimadzu Corporation, Japan) may also be used for this purpose.

For peptide synthesis through genetic engineering procedures, DNA encoding the peptide is first designed and synthesized. The design and synthesis may be accomplished, for example, by PCR techniques using a vector or the like containing the full-length hDLK-1 gene as a template and using primers which have been designed to allow synthesis of a desired DNA region. Then, the above DNA may be ligated to an appropriate vector to obtain a recombinant vector for protein expression, and this recombinant vector may be introduced into a host, such that a desired gene can be expressed therein, thereby obtaining a transformant (Molecular cloning 4th Ed. Cold Spring Harbor Laboratory Press (2012)).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis*)*,* yeast, animal cells (e.g., COS cells, CHO cells), insect cells or insects. It is also possible to use a mammal (e.g., goat) as a host. Procedures for introduction of a recombinant vector into a host are known.

Moreover, the above transformant may be cultured, and the peptide for use as an antigen may be collected from the cultured product. The term "cultured product" is intended to mean a culture supernatant, cultured cells or cultured microorganisms, or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

In the present invention, the peptide for use as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by Expressway^{™} system (Invitrogen), PURESYSTEM^{®} (Post Genome Institute Co., Ltd., Japan), TNT system^{®} (Promega), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen may be a peptide consisting of an amino acid sequence with deletion, substitution or addition of one or more amino acids in the amino acid sequence of hDLK-1 (SEQ ID NO: 2) or its partial sequence as described above. For example, it is also possible to use a peptide consisting of an amino acid sequence with deletion of one or more (preferably one or several (e.g., 1 to 10, more preferably 1 to 5)) amino acids, with substitution of other amino acids for one or more (preferably one or several (e.g., 1 to 10, more preferably 1 to 5)) amino acids, or with addition of one or more (preferably one or several (e.g., 1 to 10, more preferably 1 to 5)) other amino acids in the amino acid sequence of hDLK-1 or its partial sequence.

In the present invention, the gene to be introduced into cells or the like may be a gene encoding a hDLK-1 protein or a partial fragment thereof or a mutated protein or fragment thereof. For example, it is possible to use a gene having the nucleotide sequence shown in SEQ ID NO: 1 or a partial sequence thereof for this purpose.

Alternatively, as a gene to be introduced into cells or the like, it is also possible to use a nucleotide sequence encoding a protein with hDLK-1 activity, or a partial sequence thereof, which is hybridizable under stringent conditions with a sequence complementary to the nucleotide sequence shown in SEQ ID NO: 1.

The term "stringent conditions" refers to washing conditions after hybridization, and is intended to mean conditions where the salt (sodium) concentration in buffer is 10 to 500 mM and the temperature is 42°C to 72°C, preferably where the above salt concentration is 50 to 300 mM and the temperature is 55°C to 68°C.

For introduction of mutations into a gene, known techniques (e.g., Kunkel method or Gapped duplex method) may be used for this purpose. For example, it is possible to use a kit for mutation introduction based on site-directed mutagenesis, as exemplified by GeneTailor^{™} Site-Directed Mutagenesis System (Invitrogen), TaKaRa Site-Directed Mutagenesis System (e.g., Prime STAR^{®} Mutagenesis Basal kit, Mutan^{®}-Super Express Km; Takara Bio Inc., Japan).

### (ii) Preparation of polyclonal antibodies

The antigen prepared above is administered to a mammal for the purpose of immunization. Such a mammal is not limited in any way, and examples include rats, mice and rabbits, with mice being particularly preferred.

The amount of the antigen to be administered per animal may be determined, as appropriate, depending on the presence or absence of an adjuvant. Examples of an adjuvant include Freund's complete adjuvant (FCA), Freund's incomplete adjuvant (FIA), aluminum hydroxide adjuvant and so on. Immunization may be primarily accomplished by injection via the intravenous, footpad, subcutaneous or intraperitoneal route, etc. Moreover, the interval between immunizations is not limited in any way, and immunization may be repeated once to 10 times, preferably twice to three times, at intervals of several days to several weeks, preferably at intervals of 1 week. Further, at 3 to 7 days after the day of the final immunization, the animals are measured for their antibody titers by enzyme immunoassay (ELISA or EIA) or radioactive immunoassay (RIA), etc., and blood may be collected at the day when each animal shows the desired antibody titer, thereby obtaining antisera. In cases where antibodies are required to be purified in the above antibody collection procedures, known techniques such as salting out with ammonium sulfate, ion exchange chromatography, gel filtration chromatography, affinity chromatography and so on may be selected as appropriate or used in combination for purification purposes. Then, polyclonal antibodies in the antisera are measured for their reactivity by ELISA assay, etc.

### (iii) Preparation of monoclonal antibody

### Collection of antibody-producing cells

The anti-hDLK-1 antibody of the present invention is not limited in any way, but is preferably a monoclonal antibody.

The antigen prepared above is administered to a mammal (e.g., rat, mouse, rabbit) for the purpose of immunization. The amount of the antigen to be administered per animal may be determined, as appropriate, depending on the presence or absence of an adjuvant. Examples of an adjuvant are the same as described above. Immunization procedures are also the same as described above. Further, at 1 to 60 days, preferably 1 to 14 days, after the day of the final immunization, antibody-producing cells are collected. Antibody-producing cells may be exemplified by spleen cells, lymph node cells and peripheral blood cells, etc., with lymph node cells or spleen cells being particularly preferred.

### Cell fusion

To obtain hybridomas (antibody-producing cell lines), cell fusion is conducted between antibody-producing cells and myeloma cells. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells.

Examples of myeloma cells include mouse myeloma cell lines, as exemplified by P3-X63-Ag8.653, P3-X63-Ag8(X63), P3-X63-Ag8.U1(P3U1), P3/NS I/1-Ag4-1(NS1) and Sp2/0-Ag14(Sp2/0), etc. Myeloma cells may be selected as appropriate in consideration of their compatibility with antibody-producing cells.

Then, myeloma cells and antibody-producing cells are provided for cell fusion. For cell fusion, in a serum-free medium for animal cell culture (e.g., DMEM or RPMI-1640 medium), 1 × 10⁶ to 1 × 10⁷/mL of antibody-producing cells are mixed with 2 × 10⁵ to 2 × 10⁶/mL of myeloma cells. The ratio of antibody-producing cells to myeloma cells (antibody-producing cells:myeloma cells) is not limited in any way, but is usually set to preferably 1:1 to 10:1, more preferably 3:1. Then, fusion reaction is conducted in the presence of a cell fusion promoter. As a cell fusion promoter, it is possible to use polyethylene glycol having an average molecular weight of 1,000 to 6,000 daltons (D), etc. Alternatively, a commercially available cell fusion apparatus using electrical stimulation (e.g., electroporation) may also be used to cause cell fusion between antibody-producing cells and myeloma cells.

### Screening and cloning of hybridomas

After cell fusion, the cells are screened to select desired hybridomas. For screening, a cell suspension may be diluted as appropriate with, e.g., RPMI-1640 medium containing fetal bovine serum and then seeded on microtiter plates, and a selective medium may be added to each well, followed by culture while replacing the selective medium as appropriate. As a result, cells growing at around 14 days after the initiation of culture in the selective medium may be obtained as hybridomas.

Subsequently, the growing hybridomas are screened as to whether an antibody reactive to hDLK-1 is present in their culture supernatants. Screening of these hybridomas is not limited in any way and may be conducted in accordance with commonly used procedures. For example, aliquots of the culture supernatants contained in the wells where hybridomas have grown may be sampled and screened by ELISA, EIA and RIA, etc.

The fused cells may be cloned by limiting dilution or other techniques. An antibody strongly reactive to hDLK-1 is determined by flow cytometry or other techniques, and a hybridoma producing this antibody is selected and established as a clone.

### Collection of monoclonal antibody

For culture of the establish hybridoma and collection of a monoclonal antibody from the resulting cultured product, commonly used procedures, e.g., cell culture-based procedures or ascites formation procedures may be used for this purpose. The term "culture" is intended to mean that a hybridoma is allowed to grow in a culture dish or a culture bottle, or that a hybridoma is allowed to proliferate in the abdominal cavity of an animal as described below.

In cell culture-based procedures, the hybridoma may be cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days to obtain an antibody from its culture supernatant.

In the case of ascites formation procedures, the hybridoma may be intraperitoneally administered at about 1 × 10⁷ cells to an animal of the same species as the mammal from which myeloma cells are derived, whereby the hybridoma is allowed to proliferate in abundance. Then, its ascites is preferably collected after 2 to 3 weeks.

In cases where the antibody is required to be purified in the above antibody collection procedures, known techniques such as salting out with ammonium sulfate, ion exchange chromatography, gel filtration, affinity chromatography and so on may be selected as appropriate or used in combination for purification purposes.

### Selection of clone having antitumor activity

The anti-hDLK-1 antibody for use in the present invention is an antibody having *in vivo* antitumor activity.

As used herein, the term "antitumor activity" is intended to mean tumor cell (cancer cell) killing activity or tumor growth inhibitory activity. In the present invention, antitumor activity is preferably exemplified by tumor angiogenesis inhibitory activity. As to the type of human tumor (tumor cells) against which the anti-hDLK-1 antibody of the present invention can exert antitumor activity, examples include human tumors which have been confirmed to express hDLK-1.

The presence of *in vivo* antitumor activity may be confirmed, for example, by using a cancer-bearing mouse transplanted subcutaneously with desired tumor cells, and administering this mouse with the antibody obtained as described above. In this case, the antibody may be administered either immediately after transplantation of tumor cells (Prevention model) or after confirming that the tumor has grown to a certain volume after transplantation (Treatment model). The antibody may be administered in any manner, for example, may be administered once every 3 days at a dose of 20 mg/kg body weight via the intraperitoneal route. In the case of the Prevention model, the presence or absence of antitumor activity and the level thereof may be evaluated on the basis of tumorigenesis frequency and tumor volume. In the case of the Treatment model, the presence or absence of antitumor activity and the level thereof may be evaluated on the basis of tumor volume and tumor weight.

In the present invention, the anti-hDLK-1 antibody having *in vivo* antitumor activity is not limited in any way, but it is possible to use anti-hDLK-1 antibodies as disclosed in the following gazettes: WO2008/056833, WO2009/116670 and WO2014/054820.

In more detail, examples include anti-hDLK-1 antibodies (a) to (k) shown below, and at least one of them may be used:
(a) an antibody in which the amino acid sequences of H chain V region CDRs 1 to 3 are the amino acid sequences shown in SEQ ID NOs: 3 to 5, respectively, and the amino acid sequences of L chain V region CDRs 1 to 3 are the amino acid sequences shown in SEQ ID NOs: 6 to 8, respectively;
(b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
(c) an antibody in which the amino acid sequence of the H chain V region consists of the amino acid sequence shown in SEQ ID NO: 16 (the corresponding nucleotide sequence is SEQ ID NO: 15), and the amino acid sequence of the L chain V region consists of the amino acid sequence shown in SEQ ID NO: 18 (the corresponding nucleotide sequence is SEQ ID NO: 17), and it should be noted that CDR sequences in the H chain V region and L chain V region of this antibody are the same as those in the antibody shown in (a) above;
(d) an antibody in which the amino acid sequence of the H chain V region consists of the amino acid sequence shown in SEQ ID NO: 20 (the corresponding nucleotide sequence is SEQ ID NO: 19), and the amino acid sequence of the L chain V region consists of the amino acid sequence shown in SEQ ID NO: 22 (the corresponding nucleotide sequence is SEQ ID NO: 21), and it should be noted that CDR sequences in the H chain V region and L chain V region of this antibody are the same as those in the antibody shown in (b) above;
(e) an antibody (humanized antibody) in which the amino acid sequence of the H chain V region consists of the amino acid sequence shown in SEQ ID NO: 24 or 26 (the corresponding nucleotide sequence is SEQ ID NO: 23 or 25), and the amino acid sequence of the L chain V region consists of the amino acid sequence shown in SEQ ID NO: 28 (the corresponding nucleotide sequence is SEQ ID NO: 27), and it should be noted that CDR sequences in the H chain V region and L chain V region of this antibody are the same as those in the antibody shown in (a) above;
(f) an antibody (humanized antibody) in which the amino acid sequence of the H chain V region consists of the amino acid sequence shown in SEQ ID NO: 30, 32, 34 or 36 (the corresponding nucleotide sequence is SEQ ID NO: 29, 31, 33 or 35), and the amino acid sequence of the L chain V region consists of the amino acid sequence shown in SEQ ID NO: 46 (the corresponding nucleotide sequence is SEQ ID NO: 45), and it should be noted that CDR sequences in the H chain V region and L chain V region of this antibody are the same as those in the antibody shown in (b) above;
(g) an antibody (humanized antibody) in which the amino acid sequence of the H chain V region consists of the amino acid sequence shown in SEQ ID NO: 38, 40, 42 or 44 (the corresponding nucleotide sequence is SEQ ID NO: 37, 39, 41 or 43), and the amino acid sequence of the L chain V region consists of the amino acid sequence shown in SEQ ID NO: 46 (the corresponding nucleotide sequence is SEQ ID NO: 45), and it should be noted that CDR sequences in the H chain V region and L chain V region of this antibody are the same as those in the antibody shown in (b) above;
(h) an antibody produced by the hybridoma of Accession No. FERM BP-10899;
(i) an antibody produced by the hybridoma of Accession No. FERM BP-10707;
(j) an antibody produced by the hybridoma of Accession No. FERM BP-10900; and
(k) an antibody produced by the hybridoma of Accession No. FERM BP-11337.

With regard to the antibody shown in (f) above, the amino acid sequence shown in SEQ ID NO: 32 comprises a substitution from alanine (A) to glycine (G) at position 24 in the amino acid sequence shown in SEQ ID NO: 30,
the amino acid sequence shown in SEQ ID NO: 34 comprises a substitution from threonine (T) to lysine (K) at position 74 in the amino acid sequence shown in SEQ ID NO: 30, and
the amino acid sequence shown in SEQ ID NO: 36 comprises a substitution from alanine (A) to glycine (G) at position 24 and a substitution from threonine (T) to lysine (K) at position 74 in the amino acid sequence shown in SEQ ID NO: 30.

Likewise, with regard to the antibody shown in (g) above, the amino acid sequence shown in SEQ ID NO: 40 comprises a substitution from alanine (A) to glycine (G) at position 24 in the amino acid sequence shown in SEQ ID NO: 38,
the amino acid sequence shown in SEQ ID NO: 42 comprises a substitution from threonine (T) to lysine (K) at position 74 in the amino acid sequence shown in SEQ ID NO: 38, and
the amino acid sequence shown in SEQ ID NO: 44 comprises a substitution from alanine (A) to glycine (G) at position 24 and a substitution from threonine (T) to lysine (K) at position 74 in the amino acid sequence shown in SEQ ID NO: 38.

Such modified antibodies comprising amino acid substitutions in the antibodies (humanized antibodies) shown in (f) and (g) above are antibodies with much higher avidity (antigen binding activity), for example, such that they are capable of retaining their binding activity to cancer cells showing low antigen expression levels on the cell surface. In addition, these modified antibodies are capable of retaining their long-term stable antigen binding activity in liquid formulations and in monkey or human blood (plasma), etc.

Moreover, with regard to the antibodies shown in (h) to (k) above, the hybridoma of Accession No. FERM BP-11337 was designated as "Mouse-Mouse hybridoma BA-1-3D" and deposited on February 1, 2011, the hybridoma of Accession No. FERM BP-10707 was designated as "Mouse-Mouse hybridoma: M3-1" and deposited on October 18, 2006, the hybridoma of Accession No. FERM BP-10899 was designated as "Mouse-Mouse hybridoma DI-2-14" and deposited on August 21, 2007, and the hybridoma of Accession No. FERM BP-10900 was designated as "Mouse-Mouse hybridoma DI-6" and deposited on August 21, 2007.

Anti-hDLK-1 antibodies available for use in the present invention preferably include those capable of competing with the above various anti-hDLK-1 antibodies, as exemplified by anti-hDLK-1 antibodies binding to sites (e.g., epitopes) to which the above various anti-hDLK-1 antibodies bind (recognize), etc.

### Epitope for anti-hDLK-1 antibody

The epitope (antigenic determinant) for the anti-hDLK-1 antibody is not limited in any way as long as it is at least a part of the antigen hDLK-1, but it is preferably, for example, at least a part of a region consisting of amino acids at positions 24 to 91 (i.e., a region comprising EGF-1 to EGF-2 of hDLK-1), a region consisting of amino acids at positions 92 to 167 (i.e., a region comprising EGF-3 to EGF-4 of hDLK-1), or a region consisting of amino acids at positions 131 to 244 (i.e., a region comprising EGF-4 to EGF-6 of hDLK-1) in the amino acid sequence of hDLK-1 shown in SEQ ID NO: 2. Above all, more preferred is a region comprising EGF-1 to EGF-2 of hDLK-1. The anti-hDLK-1 antibody recognizing such a region (binding to such a region), for example, has high internalization activity into tumor cells and is therefore very useful for use in immunoconjugates as described later.

### (iv) Genetically recombinant antibodies and antibody fragments

### Genetically recombinant antibodies

A preferred embodiment of the anti-hDLK-1 antibody may be a genetically recombinant antibody. Examples of a genetically recombinant antibody include, but are not limited to, a chimeric antibody and a humanized antibody, etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for easy construction such that the thus linked antibody is obtained.

For preparation of a humanized antibody, complementarity determining regions (CDRs) from mouse antibody variable regions are grafted into human variable regions to prepare reconstituted variable regions whose framework regions (FRs) are of human origin and whose CDRs are of mouse origin (so-called CDR grafting). Then, these humanized reconstituted human variable regions are linked to human constant regions. For details of how to prepare such a humanized antibody, reference may be made to, for example, Nature, 321, 522-525 (1986); J. Mol. Biol., 196, 901-917 (1987); Queen C et al., Proc. Natl. Acad. Sci. USA, 86: 10029-10033 (1989); JP H04-502408 A (Japanese Patent No. 2828340; Queen et al.), etc. Further, the present invention may also encompass modified antibodies in which some amino acids (preferably one to several, more preferably one or two amino acids) in the H chain or L chain V region (except for CDR sequences) of the above humanized antibody are replaced with other amino acids. Such modified humanized anti-hDLK-1 antibodies may be humanized antibodies with much higher avidity (antigen binding activity), for example, such that they are capable of retaining their binding activity to cancer cells showing low antigen expression levels on the cell surface. In addition, these modified humanized anti-hDLK-1 antibodies may be capable of retaining their long-term stable antigen binding activity in liquid formulations and in monkey or human blood (plasma), etc.

The above chimeric and humanized antibodies are configured, for example, such that the N-glycoside-linked complex sugar chain in the antibody Fc region preferably has no fucose linked to N-acetylglucosamine at the reducing terminal of the sugar chain, as specifically exemplified by antibodies composed of genetically recombinant antibody molecules whose Fc region has a sugar chain in which the 1-position of the fucose is not α-liked to the 6-position of N-acetylglucosamine at the reducing terminal of the N-glycoside-linked complex sugar chain. Such an antibody allows a dramatic improvement in ADCC activity. It should be noted that this point (i.e., the characteristics of the N-glycoside-linked complex sugar chain in the antibody Fc region) is also preferred for the above polyclonal and monoclonal antibodies.

### Antibody fragments

In the present invention, a fragment of the anti-hDLK-1 antibody may also be used in combination with the anti-hDLK-1 antibody of the present invention or in place of the anti-hDLK-1 antibody of the present invention. Such an antibody fragment has binding activity to hDLK-1, as in the case of the anti-hDLK-1 antibody of the present invention (including humanized antibody, etc., except for mouse antibody), and also has *in vivo* antitumor activity.

Such an antibody fragment is intended to mean a partial region of the anti-hDLK-1 polyclonal antibody or the anti-hDLK-1 monoclonal antibody (i.e., an antibody fragment derived from the anti-hDLK-1 antibody of the present invention), and examples include Fab, Fab', F(ab')₂, Fv (variable fragment of antibody), single chain antibody (e.g., H chain, L chain, H chain V region, and L chain V region), scFv, diabody (scFv dimer), dsFv (disulfide stabilized V region), as well as peptides at least partially containing complementarity determining regions (CDRs), etc.

Fab is an antibody fragment having a molecular weight of about 50,000 and having antigen binding activity, which is composed of the N-terminal half of H chain and the full length of L chain linked via a disulfide bond, among fragments obtained by treating an antibody molecule with a protease, papain. Alternatively, Fab may also be prepared as follows: DNA encoding antibody Fab is inserted into a prokaryotic or eukaryotic expression vector, and this vector is introduced into a prokaryotic or eukaryotic organism for expression.

F(ab')₂ is an antibody fragment having a molecular weight of about 100,000 and having antigen binding activity, which is slightly larger than that composed of Fab fragments linked via disulfide bonds in the hinge region, among fragments obtained by treating an antibody molecule with a protease, pepsin. Alternatively, F(ab')₂ may also be prepared by linking Fab' fragments described later via thioether bonds or disulfide bonds.

Fab' is an antibody fragment having a molecular weight of about 50,000 and having antigen binding activity, which is obtained by cleaving the disulfide bonds in the hinge region of the above F(ab')₂. Alternatively, Fab' may also be prepared as follows: DNA encoding an antibody Fab' fragment is inserted into a prokaryotic or eukaryotic expression vector, and this vector is introduced into a prokaryotic or eukaryotic organism for expression.
scFv is an antibody fragment having antigen binding activity, which is composed of a single H chain V region (VH) and a single L chain V region (VL) linked via an appropriate peptide linker (P), i.e., a VH-P-VL or VL-P-VH polypeptide. For preparation of scFv, cDNAs encoding antibody VH and VL may be obtained to construct DNA encoding scFv, and this DNA may be inserted into a prokaryotic or eukaryotic expression vector, followed by introducing this expression vector into a prokaryotic or eukaryotic organism for expression.

Diabody is an antibody fragment composed of dimerized scFv fragments and having divalent antigen binding activity. The divalent antigen binding activity may be directed to the same antigen or to different antigens. For preparation of diabody, cDNAs encoding antibody VH and VL may be obtained to construct DNA encoding scFv such that the amino acid sequence of P has a length of 8 residues or less, and this DNA may be inserted into a prokaryotic or eukaryotic expression vector, followed by introducing this expression vector into a prokaryotic or eukaryotic organism for expression.
dsFv is an antibody fragment composed of VH and VL polypeptides, in each of which a single amino acid residue is replaced with a cysteine residue and which are linked via a disulfide bond between these cysteine residues. An amino acid residue to be replaced with a cysteine residue can be selected based on three-dimensional structure prediction of antibody according to the method reported by Reiter et al. (Protein Engineering, 7, 697-704, 1994). For preparation of dsFv, cDNAs encoding antibody VH and VL may be obtained to construct DNA encoding dsFv, and this DNA may be inserted into a prokaryotic or eukaryotic expression vector, followed by introducing this expression vector into a prokaryotic or eukaryotic organism for expression.

A CDR-containing peptide is configured to comprise at least one or more regions of VH or VL CDRs (CDRs 1 to 3). In the case of a peptide containing a plurality of CDRs, these CDRs may be linked directly or through an appropriate peptide linker. For preparation of a CDR-containing peptide, DNA encoding CDR in antibody VH or VL may be constructed, and the DNA may be inserted into a prokaryotic or eukaryotic expression vector, followed by introducing this expression vector into a prokaryotic or eukaryotic organism for expression. Alternatively, a CDR-containing peptide may also be prepared by chemical synthesis such as Fmoc (fluorenylmethyloxycarbonyl) and tBoc (t-butyloxycarbonyl) methods.

Antibody fragments for use in the present invention may be antibody fragments comprising a part or the whole of the antibody Fc region whose N-glycoside-linked complex sugar chain has no fucose linked to N-acetylglucosamine at the reducing terminal of the sugar chain, or alternatively, may be fusion proteins of the antibody fragments mentioned above with a part or the whole of the antibody Fc region whose N-glycoside-linked complex sugar chain has no fucose linked to N-acetylglucosamine at the reducing terminal of the sugar chain. Such antibody fragments allow a dramatic improvement in ADCC activity and are therefore preferred.

Specific examples of antibody fragments for use in the present invention include, but are not limited to, those comprising H chain V region CDRs 1 to 3 and L chain V region CDRs 1 to 3 in the various anti-hDLK-1 antibodies mentioned above, and those comprising the entire H chain V region and the entire the L chain V region in the various anti-hDLK-1 antibodies mentioned above.

### (v) Antibody-drug conjugate

The anti-hDLK-1 antibody and antibody fragments for use in the present invention may be in the form of conjugates with a compound having antitumor activity and/or cell killing activity. It should be noted that a product obtained when an antibody molecule or an antibody fragment molecule and a compound having antitumor activity and/or cell killing activity are each prepared in advance and then conjugated with each other is generally referred to as an immunoconjugate. Likewise, a product obtained when a protein toxin serving as a compound having antitumor activity and/or cell killing activity is ligated on its gene to a gene for an antibody or antibody fragment by gene recombination technology and allowed to be expressed as one protein (fusion protein) is generally referred to as an immunotoxin.

Examples of a compound having antitumor activity include doxorubicin, calicheamicin, mitomycin C, Auristatin E and so on. Examples of a compound having cell killing activity include saporin, ricin, pseudomonas exotoxin, diphtheria toxin and so on, with saporin and pseudomonas exotoxin being preferred for use.

Such a conjugate may be prepared in any manner, for example, by coupling an antibody to a drug via a disulfide bond or a hydrazone bond.

The anti-hDLK-1 antibody for use in the present invention is excellent in internalization activity into target tumor cells expressing hDLK-1. For this reason, when previously conjugated with a compound having antitumor activity and/or cell killing activity, the anti-hDLK-1 antibody allows such a compound to directly and highly selectively act on tumor cells. The thus obtained conjugate is very excellent in the ability of drug delivery to target tumor cells.

It should be noted that the internalization activity into cells may be evaluated as follows: an antibody is fluorescently labeled with rhodamine or the like and observed for its migratory behavior into cells and its localization therein under a fluorescence microscope, etc.

In the pharmaceutical combination of the present invention, the content of the anti-hDLK-1 antibody as an active ingredient is not limited in any way and may be determined as appropriate, but may be set to be within the range of 0.01% to 99.99% by weight, relative to the total weight of the pharmaceutical combination, and preferably set to be within the range of 0.01% to 30% by weight, more preferably 0.05% to 20% by weight, even more preferably 0.1% to 10% by weight, relative to the total weight of the pharmaceutical combination. The content of the active ingredient within the above range is sufficient for the pharmaceutical combination of the present invention to exert a therapeutic effect on cancer.

### (3) Pharmaceutical combination

The pharmaceutical combination of the present invention comprises the FGFR4 inhibitor or other form thereof and the anti-hDLK-1 antibody or a fragment thereof as active ingredients, and may further comprise other drugs for use against cancer or tumor (e.g., anticancer agents).

The pharmaceutical combination of the present invention may be administered to a subject, i.e., a human or non-human mammal (e.g., mouse, rat, rabbit, sheep, pig, cow, cat, dog, monkey) by various routes of administration, as specifically exemplified by oral administration or parenteral administration (e.g., intravenous injection, intramuscular injection, intraperitoneal injection, subcutaneous injection, intrarectal administration, percutaneous administration).

Thus, the pharmaceutical combination of the present invention may be not only used alone, but also formulated with a pharmaceutically acceptable carrier into an appropriate dosage form in a manner commonly used, depending on the intended route of administration. Moreover, the pharmaceutical combination of the present invention also includes the form of an antibody-drug conjugate where a substance which inhibits or suppresses tyrosine kinase activity is linked to an anti-DLK-1 antibody.

Dosage forms for oral formulations may be exemplified by tablets, powders, fine granules, granules, coated tablets, capsules, solutions for internal use, suspensions, emulsions, syrups and troches, etc., while dosage forms for parenteral formulations may be exemplified by injections (including drops), inhalants, ointments, nose drops, and liposomes, etc.

Examples of carriers which may be used to formulate these formulations include commonly used excipients, binders, disintegrants, lubricants, colorants, and correctives, as well as optionally stabilizers, emulsifiers, absorbefacients, surfactants, pH adjusters, antiseptics, antioxidants, extenders, humectants, surface active agents, dispersants, buffering agents, preservatives, solvent aids, and soothing agents, etc., which may be blended with known ingredients available for use as source materials for pharmaceutical formulations and then formulated in a standard manner.

Non-toxic ingredients available for this purpose may be exemplified by animal and vegetable oils such as soybean oil, beef tallow, and synthetic glycerides; hydrocarbons such as liquid paraffin, squalane, and hard paraffin; ester oils such as octyldodecyl myristate, and isopropyl myristate; higher alcohols such as cetostearyl alcohol, and behenyl alcohol; silicone resin; silicone oil; surfactants such as polyoxyethylene fatty acid esters, sorbitan fatty acid esters, glycerin fatty acid esters, polyoxyethylene sorbitan fatty acid esters, polyoxyethylene hydrogenated castor oil, and polyoxyethylene-polyoxypropylene block copolymers; water-soluble polymers such as hydroxyethylcellulose, polyacrylic acid, carboxyvinyl polymers, polyethylene glycol, polyvinylpyrrolidone, and methylcellulose; lower alcohols such as ethanol, and isopropanol; polyhydric alcohols (polyols) such as glycerin, propylene glycol, dipropylene glycol, sorbitol, and polyethylene glycol; sugars such as glucose, and sucrose; inorganic powders such as silicic anhydride, magnesium aluminum silicate, and aluminum silicate; inorganic salts such as sodium chloride, and sodium phosphate; and purified water. These ingredients may be in the form of salts or hydrates thereof.

Preferred examples of excipients include lactose, fructose, corn starch, sucrose, glucose, mannitol, sorbit, crystalline cellulose, and silicon dioxide, etc. Preferred examples of binders include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, polypropyleneglycol-polyoxyethylene block polymers, and meglumine, etc. Preferred examples of disintegrants include starch, agar, gelatin powder, crystalline cellulose, calcium carbonate, sodium bicarbonate, calcium citrate, dextrin, pectin, and carboxymethylcellulose calcium, etc. Preferred examples of lubricants include magnesium stearate, talc, polyethylene glycol, silica, and hydrogenated vegetable oils, etc. Preferred examples of colorants include those approved for addition to pharmaceutical products. Preferred examples of correctives include cocoa powder, menthol, aromatic powder, peppermint oil, borneol, and cinnamon powder, etc. These ingredients may be in the form of salts or hydrates thereof.

The dose of the pharmaceutical combination of the present invention may generally be determined extensively as appropriate for the age and body weight of a subject (patient) to be administered, the type and progression of disease, the route of administration, the frequency of administration (per day), the period of administration, etc., in consideration of the ratio of the active ingredients incorporated into the formulation. Moreover, in the pharmaceutical combination of the present invention, the active ingredients, i.e., the FGFR4 inhibitor or other form thereof and the anti-hDLK-1 antibody or a fragment thereof may be administered substantially at the same time or administered sequentially in the order in which one is ahead of the other, without being limited thereto. In addition, these administrations are not limited in any way, and the amounts contained in the pharmaceutical combination may be administered at once or continuously.

A detailed explanation will be given below for the case where the pharmaceutical combination of the present invention is used as a parenteral formulation or an oral formulation.

For use as a parenteral formulation, the pharmaceutical combination of the present invention may usually be formulated into any dosage form. In the case of various types of injections, for example, they may be provided in the form of unit dose ampules or multi-dose containers or as freeze-dried powders which are dissolved again in a diluent before use. Such a parenteral formulation may comprise not only the FGFR4 inhibitor or other form thereof and the anti-hDLK-1 antibody or a fragment thereof serving as active ingredients, but also various known excipients and/or additives as appropriate for each dosage form as long as the effect of the above active ingredients is not impaired. Examples of excipients and/or additives include water, glycerol, propylene glycol, and aliphatic polyalcohols such as polyethylene glycol, etc., in the case of various types of injections.

The dose (daily dose) of such a parenteral formulation is not limited in any way. For example, in the case of various types of injections, the dose may generally be set such that the FGFR4 inhibitor or other form thereof and the anti-hDLK-1 antibody or a fragment thereof serving as active ingredients can be taken in an amount of 0.01 to 1000 mg, 0.05 to 500 mg or 0.1 to 50 mg, per kg body weight of a subject to be applied (e.g., a test subject, a patient), or alternatively, can be taken in an amount of 0.5 to 20 mg or can be taken in an amount of 1 to 10 mg.

For use as an oral formulation, the pharmaceutical combination of the present invention may usually be formulated into any dosage form among those mentioned above, or alternatively, may be formulated into a freeze-dried product which is dissolved again before use. Such an oral formulation may comprise not only the FGFR4 inhibitor or other form thereof and the anti-hDLK-1 antibody or a fragment thereof serving as active ingredients, but also various known excipients and/or additives as appropriate for each dosage form as long as the effect of the above active ingredients is not impaired. Examples of excipients and/or additives include binders (e.g., syrup, gum arabic, gelatin, sorbitol, tragacanth, polyvinylpyrrolidone), fillers (e.g., lactose, sugar, corn starch, potato starch, calcium phosphate, sorbitol, glycine), lubricants (e.g., magnesium stearate, talc, polyethylene glycol, silica), disintegrants (e.g., various types of starches), and wetting agents (e.g., sodium lauryl sulfate), etc.

The dose (daily dose) of such an oral formulation may generally be set such that the FGFR4 inhibitor or other form thereof and the anti-hDLK-1 antibody or a fragment thereof serving as active ingredients can be taken in an amount of 0.05 to 5000 mg, 0.1 to 1000 mg or 0.1 to 100 mg, per kg body weight of a subject to be applied (e.g., a test subject, a patient), or alternatively, can be taken in an amount of 0.5 to 50 mg or can be taken in an amount of 1 to 10 mg. Moreover, the ratio of the active ingredients incorporated into the oral formulation is not limited in any way and may be set as appropriate in consideration of the frequency of administration per day, etc.

The pharmaceutical combination of the present invention is capable of exerting a more potent and sustained antitumor effect than existing FGFR4-selective tyrosine kinase inhibitors in the treatment of various types of cancers, particularly cancers where FGFR4 is expressed and/or the tyrosine kinase activity of FGFR4 is observed, as exemplified by hepatocellular carcinoma. For example, the pharmaceutical combination of the present invention allows suppression of cancer cell proliferation or allows tumor reduction or disappearance even after completing the administration of the pharmaceutical combination. The pharmaceutical combination of the present invention is very useful in terms of being capable of exerting a sufficient therapeutic effect on cancer patients who could not have been expected to experience any therapeutic effect (or who have been nonresponders) when administered with the above existing tyrosine kinase inhibitors alone. Moreover, the pharmaceutical combination of the present invention may have the effect of delaying, arresting or reducing the onset of at least one clinical symptom associated with diseases (the above cancers), or may relax or improve or stabilize at least one physical parameter (e.g., body weight reduction) associated with diseases (the above cancers), also including those which are not recognizable by patients.

### 3. Pharmaceutical composition

In addition to the pharmaceutical combination for the treatment of cancer described in 2 above, the present invention also includes inventions of pharmaceutical compositions shown in (i) to (iv) below:
(i) a pharmaceutical composition for the treatment of cancer, comprising an antibody against human DLK-1, which has *in vivo* antitumor activity, or an antibody fragment derived from the antibody,
   wherein the pharmaceutical composition is used in combination with a substance which inhibits or suppresses the tyrosine kinase activity of FGFR4, or a prodrug thereof, or a pharmacologically acceptable salt thereof, or a hydrate or solvate thereof;
(ii) a pharmaceutical composition for the treatment of cancer, comprising a substance which inhibits or suppresses the tyrosine kinase activity of FGFR4, or a prodrug thereof, or a pharmacologically acceptable salt thereof, or a hydrate or solvate thereof,
   wherein the pharmaceutical composition is used in combination with an antibody against human DLK-1, which has *in vivo* antitumor activity, or an antibody fragment derived from the antibody;
(iii) a pharmaceutical composition for the treatment of cancer, comprising an antibody against human DLK-1, which has *in vivo* antitumor activity, or an antibody fragment derived from the antibody,
   wherein the treatment comprises administering a substance which inhibits or suppresses the tyrosine kinase activity of FGFR4, or a prodrug thereof, or a pharmacologically acceptable salt thereof, or a hydrate or solvate thereof; and
(iv) a pharmaceutical composition for the treatment of cancer, comprising a substance which inhibits or suppresses the tyrosine kinase activity of FGFR4, or a prodrug thereof, or a pharmacologically acceptable salt thereof, or a hydrate or solvate thereof,
   wherein the treatment comprises administering an antibody against human DLK-1, which has *in vivo* antitumor activity, or an antibody fragment derived from the antibody.

For details about the FGFR4 inhibitor or other form thereof and the anti-hDLK-1 antibody or a fragment thereof in the pharmaceutical compositions shown in (i) to (iv) above (e.g., their content, their dosage regimen, subjects to be administered therewith), and for details about cancers to be treated and the "tumor" in the antitumor activity of the anti-hDLK-1 antibody or a fragment thereof, etc., reference may be made as appropriate to the descriptions about the pharmaceutical combination for the treatment of cancer given in 2 above.

### 4. Kit

The present invention can also be provided in the form of a kit for the treatment of cancer, comprising an FGFR4 inhibitor or other form thereof and an anti-hDLK-1 antibody or a fragment thereof as constituent elements. For details about cancers to be treated, etc., reference may be made as appropriate to the descriptions about the pharmaceutical combination for the treatment of cancer given in 2 above.

In such a kit, the FGFR4 inhibitor or other form thereof and the anti-hDLK-1 antibody or a fragment thereof may be provided, for example, in a dissolved state in consideration of their stability (storage quality) and easiness of use, etc.

Such a kit may also comprise other constituent elements, as appropriate, in addition to the FGFR4 inhibitor or other form thereof and the anti-hDLK-1 antibody or a fragment thereof. For example, the kit may further comprise an antibody labeling substance, or alternatively, an immobilized reagent in which an antibody or a labeled product thereof is immobilized. The antibody labeling substance is intended to mean a substance labeled with an enzyme, a radioisotope, a fluorescent compound, a chemiluminescent compound or the like. Moreover, the kit may also comprise various buffers, sterilized water, various cell culture vessels, various reaction vessels (e.g., Eppendorf tubes), a blocking agent (e.g., bovine serum albumin (BSA), skimmed milk, goat serum or other serum components), a detergent, a surfactant, various plates, an antiseptic (e.g., sodium azide), an instruction manual for experimental operations (manufacturer's instructions) and so on.

The kit is required to comprise at least the above FGFR4 inhibitor or other form thereof and the above anti-hDLK-1 antibody or fragment thereof as constituent elements. Thus, the kit may be configured to comprise the constituent elements essential for the treatment of cancer, either all together or separately from each other, without being limited thereto.

The present invention will be further described in more detail by way of the following examples, although the present invention is not limited only to these examples.

### [Example 1]

Hep3B cells (1 × 10⁶ cells) were transplanted subcutaneously into the right flank of NOD-SCID mice. At the stage where the tumor volume reached 250 to 350 mm³, these mice were divided into the following 8 groups of 6 mice each, and administered from the same day:
the Vehicle group;
the group receiving HuBA-1-3D (1 mg/kg);
the group receiving H3B-6527 (50 mg/kg);
the group receiving FGF-401 (10 mg/kg);
the group receiving BLU-554 (30 mg/kg);
the group receiving HuBA-1-3D (1 mg/kg) + H3B-6527 (50 mg/kg);
the group receiving HuBA-1-3D (1 mg/kg) + FGF-401 (10 mg/kg); and
the group receiving HuBA-1-3D (1 mg/kg) + BLU-554 (30 mg/kg).

HuBA-1-3D was intraperitoneally administered, while FGFR4 inhibitors (H3B-6527, FGF-401 and BLU-554) were orally administered. At 48 hours after administration, three mice in each group were sacrificed to excise their tumors, and the tumors were fixed in 10% formalin and then embedded in paraffin to prepare sections for immunostaining. In addition, tissue extracts were prepared for Western blotting.

Cancer cell proliferation and apoptosis in the Hep3B tumor at 48 hours after administration were analyzed by immunostaining in which Ki-67 or cleaved caspase-3 expression was used as an indicator. The results obtained are shown in Figures 1 to 3.
Figure 1 shows Ki-67 staining images (upper) and cleaved caspase-3 staining images (lower) in the Vehicle group, the group receiving HuBA-1-3D (1 mg/kg), the group receiving H3B-6527 (50 mg/kg) and the group receiving HuBA-1-3D (1 mg/kg) + H3B-6527 (50 mg/kg). In the Hep3B xenograft tumor in the Vehicle group, proliferating cancer cells with Ki-67-stained nuclei were observed throughout the tumor. In the group receiving HuBA-1-3D (1 mg/kg) and the group receiving H3B-6527 (50 mg/kg), there was no change in the proportion of cancer cells stained with Ki-67 when compared to the Vehicle group. In contrast, when HuBA-1-3D (1 mg/kg) and H3B-6527 (50 mg/kg) were administered in combination, cancer cells stained with Ki-67 in the Hep3B xenograft tumor were significantly reduced in their proportion. As to apoptosis of cancer cells, apoptotic cells stained with cleaved caspase-3 were not detected in the Vehicle group and the group receiving H3B-6527 (50 mg/kg), whereas apoptosis of cancer cells was detected over a wide area of the tumor in the group receiving HuBA-1-3D (1 mg/kg). In the tumor in the group receiving HuBA-1-3D (1 mg/kg) + H3B-6527 (50 mg/kg), apoptosis of cancer cells was also detected over a wide area of the tumor.

Likewise, Figure 2 shows the results obtained upon combined administration of FGF-401 (10 mg/kg) and HuBA-1-3D (1 mg/kg), while Figure 3 shows the results obtained upon combined administration of BLU-554 (30 mg/kg) and HuBA-1-3D (1 mg/kg). In Figures 2 and 3, the same photographs as shown in Figure 1 were used for the Vehicle group and the group receiving HuBA-1-3D (1 mg/kg).

In the group receiving FGF-401 (10 mg/kg) alone and the group receiving BLU-554 (30 mg/kg) alone, there was no change in the proportion of cancer cells stained with Ki-67 when compared to the Vehicle group. In contrast, in the group receiving FGF-401 (10 mg/kg) or BLU-554 (30 mg/kg) in combination with HuBA-1-3D (1 mg/kg), cancer cells stained with Ki-67 were significantly reduced in their proportion. Moreover, as to apoptosis of cancer cells, apoptotic cells stained with cleaved caspase-3 were not detected in the group receiving FGF-401 (10 mg/kg) alone and the group receiving BLU-554 (30 mg/kg) alone, whereas apoptosis of cancer cells was detected over a wide area of the tumor in the group receiving FGF-401 (10 mg/kg) or BLU-554 (30 mg/kg) in combination with HuBA-1-3D (1 mg/kg).

The results obtained above indicated that when administered in combination with HuBA-1-3D (1 mg/kg), three agents (H3B-6527, FGF-401 and BLU-554) used as FGFR4-selective inhibitors all showed a synergistic effect on the inhibition of cancer cell proliferation in the Hep3B xenograft tumor, and further indicated that apoptosis of cancer cells was induced over a wide area of the tumor in the groups receiving their combined administration.

### [Example 2]

Subsequently, apoptosis (cleaved caspase-3 and Cleaved PARP) and cancer cell proliferation (cyclin B 1) in the Hep3B xenograft tumor upon administration of each agent were analyzed by Western blotting. The results obtained are shown in Figure 4.

In the Hep3B tumor at 48 hours after administration, the expression of apoptosis markers (Cleaved caspase-3 and cleaved PARP) was strongly induced in one of the three animals in the group receiving HuBA-1-3D (1 mg/kg), thus indicating that HuBA-1-3D administration induced cell death of the Hep3B tumor. On the other hand, in the groups receiving FGFR4 inhibitors (H3B-6527, FGF-401 and BLU-554), apoptosis markers (Cleaved caspase-3 and cleaved PARP) were induced in none of the three animals administered in each of the groups (a) H3B-6527, (b) FGF-401 and (c) BLU-554. In the groups receiving FGFR4 inhibitors in combination with HuBA-1-3D, apoptosis was induced in all the three animals in each group.

As to the expression of cyclin B1 serving as a cell proliferation marker, in the group receiving HuBA-1-3D (1 mg/kg), a reduction in the expression of cyclin B1 was observed in one animal where apoptosis was induced, but the remaining two animals showed the same expression level of cyclin B1 as in the Vehicle group. In the groups receiving three FGFR4 inhibitors alone, H3B-6527, FGF-401 and BLU-554 all showed no difference in the expression level of cyclin B1 when compared to the Vehicle group. In contrast, in the groups receiving FGFR4 inhibitors in combination with HuBA-1-3D (1 mg/kg), the expression level of cyclin B1 was reduced in all the three animals in each group.

The results obtained above indicated that when administered in combination with HuBA-1-3D (1 mg/kg), three agents (H3B-6527, FGF-401 and BLU-554) used as FGFR4-selective inhibitors all showed a synergistic effect on the inhibition of cancer cell proliferation in the Hep3B xenograft tumor, and further indicated that apoptosis of cancer cells was induced in the groups receiving their combined administration.

### [Example 3]

### <Study of antitumor effect in human hepatic cancer cell line Hep3B xenograft therapeutic model upon combined administration of HuBA-1-3D antibody and FGFR4 small molecule inhibitor (H3B-6527)>

The antitumor effect obtained upon combined administration of HuBA-1-3D antibody and H3B-6527 serving as a small molecule inhibitor against FGFR4 (Cancer Res. 2017 Dec 15; 77(24):6999-7013) was studied in a xenograft therapeutic model based on the human hepatic cancer cell line Hep3B.
(1) Hep3B cells were transplanted subcutaneously into the right flank of NOD-scid mice (Day 0). At the stage where the mean tumor volume reached around 100 mm³ (Day 14), the mice were divided into the following 6 groups, and administered from the same day for 11 days with HuBA-1-3D at a frequency of twice a week (Days 14, 18, 22 and 25) and/or with H3B-6527 twice a day (BID) and five times a week in a cycle of administration for 5 days and withdrawal for 2 days (Days 14, 15, 16, 17, 18, 21, 22, 23, 24 and 25):
   the Vehicle group (N = 8)
   the group receiving H3B-6527 (50 mg/kg body weight, twice a day) (N = 8);
   the group receiving HuBA-1-3D (0.3 mg/kg body weight) (N = 8);
   the group receiving HuBA-1-3D (1 mg/kg body weight) (N = 8);
   the group receiving H3B-6527 (50 mg/kg body weight) and HuBA-1-3D (0.3 mg/kg body weight) (N = 8); and
   the group receiving H3B-6527 (50 mg/kg body weight) and HuBA-1-3D (1 mg/kg body weight) (N = 8).

The tumor volume was measured at a frequency of twice a week, and an animal whose tumor volume reached 1,500 mm³ was excluded from observation.

The tumor volume at 32 days after transplantation of the cancer cells (on Day 32, i.e., at 7 days after completion of administration) was 1377.0 ± 239.1 mm³ in the Vehicle group (N = 8), whereas it was 647.8 ± 146.2 mm³ in the group receiving H3B-6527 (50 mg/kg) (N = 8, T/C = 47%, *P<0.05), 1478.4 ± 333.5 mm³ in the group receiving HuBA-1-3D (0.3 mg/kg) antibody (N = 7), 204.5 ± 173.0 mm³ in the group receiving HuBA-1-3D (1 mg/kg) antibody (N = 8, T/C = 14.9%, *P<0.05), 473.7 ± 81.6 mm³ in the group receiving H3B-6527 (50 mg/kg) + HuBA-1-3D (0.3 mg/kg) (N = 8, T/C = 34.4%, *P<0.05), and 17.0 ± 24.3 mm³ in the group receiving H3B-6527 + HuBA-1-3D (1 mg/kg) (N = 8, T/C = 1.2%, ^{∗}P<0.05). Thus, when compared to the tumor volume in the Vehicle group, a statistically significant antitumor effect was observed in the group receiving H3B-6527 (50 mg/kg), the group receiving HuBA-1-3D (1 mg/kg), the group receiving HuBA-1-3D (0.3 mg/kg) + H3B-6527 (50 mg/kg) and the group receiving HuBA-1-3D (1 mg/kg) + H3B-6527 (50 mg/kg).

When the tumor volume was compared between the group receiving H3B-6527 + HuBA-1-3D and the groups receiving them alone, the tumor volume was significantly smaller in the group receiving H3B-6527 + HuBA-1-3D (1 mg/kg) than in each of the group receiving H3B-6527 and the group receiving HuBA-1-3D (1 mg/kg) (^{∗∗}P<0.05 by Tukey test). In the group receiving H3B-6527 + HuBA-1-3D (1 mg/kg), tumor regression further progressed after the final day of administration (Day 25), and tumor disappearance was observed in 5 of the 8 animals on Day 32.

When the tumor volume was compared among the three groups, i.e., the group receiving H3B-6527 (50 mg/kg), the group receiving HuBA-1-3D (1 mg/kg) and the group receiving H3B-6527 (50 mg/kg) + HuBA-1-3D (1 mg/kg), the group receiving H3B-6527 (50 mg/kg) + HuBA-1-3D (1 mg/kg) showed a significant antitumor effect (**P<0.05 by Tukey test) when compared to each of the groups receiving them alone. Moreover, in the group receiving H3B-6527 + HuBA-1-3D, tumor reduction was observed in all the 8 animals when compared to their tumor volume data on Day 14, and tumor disappearance was observed in 5 of these 8 animals, so that a potent antitumor effect was observed upon combined administration.

The results obtained above are shown in Figure 5A.

(2) Further, the time course of tumor volume was observed until Day 38 (i.e., until 13 days after the final day of administration (Day 25)) in the group receiving H3B-6527, the group receiving HuBA-1-3D (1 mg/kg) and the group receiving H3B-6527 + HuBA-1-3D (1 mg/kg). As a result, the tumor volume was 1222.0 ± 326.5 mm³ in the group receiving H3B-6527 (N = 8) and 361.4 ± 320.4 mm³ in the group receiving HuBA-1-3D (1 mg/kg) (N = 8), whereas the tumor volume was 22.1 ± 31.9 mm³ in the group receiving H3B-6527 + HuBA-1-3D (1 mg/kg) (N = 8), thus indicating that the tumor volume was significantly smaller in the group receiving H3B-6527 + HuBA-1-3D than in each of the groups receiving them alone (*P<0.05 by Tukey)

The results obtained above are shown in Figure 5B.

(3) On Day 38 (i.e., at 13 days after the final day of administration (Day 25)), mice in the group receiving H3B-6527 (50 mg/kg), the group receiving HuBA-1-3D (1 mg/kg) and the group receiving H3B-6527 (50 mg/kg) + HuBA-1-3D (1 mg/kg) were sacrificed to excise their tumors. In the group receiving H3B-6527 (50 mg/kg) and the group receiving HuBA-1-3D (1 mg/kg), all the 8 animals in each group were confirmed to have tumor, whereas tumor disappearance was observed in 5 of the 8 animals in the group receiving H3B-6527 (50 mg/kg) + HuBA-1-3D (1 mg/kg). The tumor weight was 1.010 ± 0.260 g in the group receiving H3B-6527 (50 mg/kg) (N = 8), 0.262 ± 0.338 g in the group receiving HuBA-1-3D (1 mg/kg) (N = 8), and 0.003 ± 0.005 g in the group receiving H3B-6527 (50 mg/kg) + HuBA-1-3D (1 mg/kg) (N = 8), thus indicating that the tumor weight was also significantly smaller in the group receiving H3B-6527 + HuBA-1-3D than in each of the groups receiving them alone (^{∗}P<0.05 by Tukey), so that the enhanced antitumor effect was confirmed upon combined administration of HuBA-1-3D and H3B-6527.

The results obtained above are shown in Figure 5C.

### [Example 4]

### <Study of antitumor effect in human hepatic cancer cell line Hep3B xenograft therapeutic model upon combined administration of HuBA-1-3D antibody and FGFR4 small molecule inhibitor (FGF-401)>

Then, in an attempt to further verify the enhancing effect on antitumor activity upon combined administration of HuBA-1-3D antibody and FGFR4 inhibitor (FGF-401), the antitumor effect obtained upon combined administration of FGF-401 and HuBA-1-3D was studied in a xenograft therapeutic model based on the human hepatic cancer cell line Hep3B, wherein FGF-401 inhibits FGFR4-specific tyrosine kinase activity by specifically binding to cysteine at amino acid position 552 of the ATP-binding region present in the region located at amino acid positions 545 to 562 of FGFR4 (Mol. Cancer Ther. 2019 Dec; 18(12):2194-2206), as in the case of H3B-6527.

Hep3B cells (1 × 10⁶ cells) were transplanted subcutaneously into the right flank of NOD-SCID mice (Day 0). At the stage where the mean tumor volume exceeded 100 mm³ (Day 14), the mice were divided into the following 6 groups, and administered from the same day:
the Vehicle group (N = 8);
the group receiving FGF-401 (10 mg/kg body weight, twice a day) (N = 8);
the group receiving HuBA-1-3D (0.3 mg/kg body weight) (N = 8);
the group receiving HuBA-1-3D (1 mg/kg body weight) (N = 8);
the group receiving FGF-401 (10 mg/kg body weight) and HuBA-1-3D (0.3 mg/kg body weight) (N = 8); and
the group receiving FGF-401 (10 mg/kg body weight) and HuBA-1-3D (1 mg/kg body weight) (N = 8).

HuBA-1-3D was intraperitoneally administered once a day 4 times in total on Days 14, 17, 21 and 24, while FGF-401 was orally administered twice a day 10 times in total on Days 14, 15, 16, 17, 18, 21, 22, 23, 24 and 25. The combined administration of HuBA-1-3D and FGF-401 was performed 4 times in total on Days 14, 17, 21 and 24. In each group, a mouse whose tumor volume exceeded 1,500 mm³ was sacrificed at that point of time.

At 28 days after transplantation of the cancer cells (Day 0) (on Day 28, i.e., at 3 days after the final day of administration (Day 25)), a significant antitumor effect was observed in all the tested groups (*P<0.05 by Dunnett) when compared to the tumor volume in the Vehicle group (N = 8). The tumor volume in the Vehicle group was 684.7 ± 265.7 mm³, whereas the tumor volume was 388.9 ± 94.8 mm³ in the group receiving FGF-401 (10 mg/kg) (T/C = 56.8%, *P<0.05), 456.3 ± 232.9 mm³ in the group receiving HuBA-1-3D (0.3 mg/kg) (T/C = 66.6%, ^{∗}P<0.05), 53.5 ± 57.5 mm³ in the group receiving HuBA-1-3D (1 mg/kg) (T/C = 7.8%, *P<0.05), 180.8 ± 109.0 mm³ in the group receiving FGF-401 (10 mg/kg) + HuBA-1-3D (0.3 mg/kg) (T/C = 26.4%, *P<0.05), and 14.1 ± 21.4 mm³ in the group receiving FGF-401 (10 mg/kg) + HuBA-1-3D (1 mg/kg) (T/C = 2.1%, *P<0.05).

When the tested groups were compared in all combinations by the Tukey's multiple test, the group receiving FGF-401 (10 mg/kg) + HuBA-1-3D (0.3 mg/kg) and the group receiving FGF-401 (10 mg/kg) + HuBA-1-3D (1 mg/kg) were each found to show a significant antitumor effect (P<0.05) when compared to the group receiving FGF-401 (10 mg/kg), while the group receiving FGF-401 (10 mg/kg) + HuBA-1-3D (0.3 mg/kg) was also found to show a significant antitumor effect (P<0.05) when compared to the group receiving HuBA-1-3D (0.3 mg/kg). In the group receiving FGF-401 (10 mg/kg) + HuBA-1-3D (1 mg/kg), the antitumor effect was further enhanced (P<0.05) when compared to the group receiving FGF-401 (10 mg/kg) + HuBA-1-3D (0.3 mg/kg), so that the enhanced antitumor effect was observed upon combined administration of HuBA-1-3D and FGF-401.

Further, on Day 35 (i.e., at 10 days after the final day of administration (Day 25)), the group receiving FGF-401 (10 mg/kg), the group receiving HuBA-1-3D (1 mg/kg), the group receiving FGF-401 (10 mg/kg) + HuBA-1-3D (0.3 mg/kg) and the group receiving FGF-401 (10 mg/kg) + HuBA-1-3D (1 mg/kg) were found to show a significant antitumor effect (P<0.05 by Dunnett) when compared to the tumor volume in the Vehicle group (N = 8).

The results obtained above are shown in Figure 6.

When the tested groups were compared in all combinations by the Tukey's multiple test, the group receiving FGF-401 (10 mg/kg) + HuBA-1-3D (0.3 mg/kg) and the group receiving FGF-401 (10 mg/kg) + HuBA-1-3D (1 mg/kg) were each found to show a significant antitumor effect (P<0.05 by tukey) when compared to the group receiving FGF-401 (10 mg/kg). The group receiving FGF-401 (10 mg/kg) + HuBA-1-3D (0.3 mg/kg) was also found to show a significant antitumor effect (P<0.05 by tukey) when compared to the group receiving HuBA-1-3D (0.3 mg/kg), so that the enhanced antitumor effect was observed upon combined use of FGF-401 and HuBA-1-3D. In particular, the combined administration of FGF-401 (10 mg/kg) + HuBA-1-3D (1 mg/kg) was found to provide a significantly high antitumor effect, because 7 of the 8 animals showed tumor disappearance, and only one animal was confirmed to have tumor of 86.2 mm³.

### [Example 5]

### <Study of antitumor effect in human hepatic cancer cell line Hep3B xenograft therapeutic model upon combined administration of HuBA-1-3D antibody and FGFR4 small molecule inhibitor (BLU-554)>

In an attempt to further verify the enhancing effect on antitumor activity upon combined administration of HuBA-1-3D antibody and FGFR4 inhibitor as in the case of the above examples, the antitumor effect obtained upon combined administration of BLU-554 and HuBA-1-3D was studied in a xenograft therapeutic model based on the human hepatic cancer cell line Hep3B, wherein BLU-554 inhibits FGFR4-specific tyrosine kinase activity by specifically binding to cysteine at amino acid position 552 of the ATP-binding region present in the region located at amino acid positions 545 to 562 of FGFR4 (Cancer Discov. 2019 Dec; 9(12): 1696-1707), as in the case of H3B-6527 and FGF-401.

Hep3B cells (1 × 10⁶ cells) were transplanted (Day 0), and at the point of time when the mean tumor volume exceeded 100 mm³ (Day 14), animals were divided into the following 4 groups and administered from the same day:
the Vehicle group (N = 8);
the group receiving BLU-554 (30 mg/kg body weight, twice a day) (N = 8);
the group receiving HuBA-1-3D (1 mg/kg body weight) (N = 8); and
the group receiving BLU-554 (30 mg/kg body weight) and HuBA-1-3D (1 mg/kg body weight) (N = 8).

HuBA-1-3D was intraperitoneally administered once a day 4 times in total on Days 14, 17, 21 and 24, while BLU-554 was orally administered twice a day 10 times in total on Days 14, 15, 16, 17, 18, 21, 22, 23, 24 and 25. The combined administration of HuBA-1-3D and BLU-554 was performed 4 times in total on Days 14, 17, 21 and 24. In each group, a mouse whose tumor volume exceeded 1,500 mm³ was sacrificed at that point of time.

On Day 38 (i.e., at 13 days after the final day of administration (Day 25)), the mean tumor volume in the Vehicle group (N = 7) was 1082.1 ± 296.7 mm³, whereas the mean tumor volume was 688.8 ± 562.4 mm³ in the group receiving BLU-554 (30 mg/kg) (N = 8) (T/C = 63.7%), 127.9 ± 95.0 mm³ in the group receiving HuBA-1-3D (1 mg/kg) (N = 8) (T/C = 11.8%, *P<0.05 by Dunnett), and 56.0 ± 114.5 mm³ in the group receiving HuBA-1-3D (1 mg/kg) + BLU-554 (30 mg/kg) (N = 7) (T/C = 5.2%, ^{∗}P<0.05 by Dunnett), thus indicating that the highest antitumor effect was observed in the group receiving HuBA-1-3D (1 mg/kg) + BLU-554 (30 mg/kg).

Further, the group receiving HuBA-1-3D (1 mg/kg) and the group receiving HuBA-1-3D (1 mg/kg) + BLU-554 (30 mg/kg) were observed for their tumor growth until Day 52 (i.e., until 27 days after the final day of administration (Day 25)). The tumor volume in the group receiving HuBA-1-3D (1 mg/kg) (N = 7) reached 499.4 ± 449.4 mm³, whereas in the group receiving HuBA-1-3D (1 mg/kg) + BLU-554 (30 mg/kg) (N = 7), tumor growth was continuously suppressed in 6 animals although tumor growth was observed in one animal, and the mean tumor volume on Day 52 was 198.4 ± 388.5 mm³, so that the enhanced antitumor effect was observed upon combined administration of HuBA-1-3D (1 mg/kg) and BLU-554 (30 mg/kg).

The results obtained above are shown in Figure 7.

Currently, in the case of hepatocellular carcinoma, five types of inhibitors whose target molecule is FGFR4 are in clinical development stage (Cells. 2019, 8, 536). Among these five types of inhibitors, three types (FGF401, H3B-6527 and BLU-554) are small molecule TKIs (tyrosine kinase inhibitors), one type is an anti-FGFR4 antibody (U3-1784), and another one type is an FGFR4 antisense oligonucleotide. These three types of FGFR4 small molecule TKIs each have a mechanism of action to specifically bind to Cys552 in the ATP-binding region specific for FGFR4 and thereby inhibit the kinase activity. In this study, these three types of FGFR4-selective small molecule tyrosine kinase inhibitors were examined for their antitumor effect when administered in combination with HuBA-1-3D antibody by using the Hep3B xenograft therapeutic model. As a result, these three types of FGFR4-selective inhibitors were each confirmed to exert a significantly high antitumor effect, particularly a tumor regression effect, when administered in combination with HuBA-1-3D antibody.

### [Example 6]

### <Study of antitumor effect in human hepatic cancer cell line Hep3B xenograft therapeutic model upon combined administration of HuBA-1-3D antibody and FGFR1/FGFR2/FGFR3-selective small molecule inhibitor (CH518324/Debio1347)>

Then, in an attempt to verify whether the enhancing effect on antitumor effect upon combined administration of HuBA-1-3D antibody and FGFR4 inhibitor is specific for combination with the FGFR4 inhibitor, CH518324/Debio1347 (Mol Cancer Ther (2014); 13(11); 2547-58.), which is a selective small molecule tyrosine kinase inhibitor against FGFR1/FGFR2/FGFR3, and HuBA-1-3D antibody were studied for their combined effect. CH518324/Debio1347 is a FGFR-selective tyrosine kinase inhibitor that can be orally administered, and its tyrosine kinase inhibitory activity (IC₅₀) against four types of FGFR (i.e., FGFR1, FGFR2, FGFR3 and FGFR4) has been reported to be 9.3 nM, 7.6 nM, 22 nM and 290 nM, respectively (Mol Cancer Ther (2014); 13(11); 2547-58).

Hep3B cells (1 × 10⁶ cells) were transplanted (Day 0), and at the point of time when the mean tumor volume exceeded 100 mm³ (Day 14), the mice were divided into the following 6 groups and administered from the same day:
the Vehicle group (N = 8);
the group receiving CH518324/Debio1347 (50 mg/kg body weight, once a day) (N = 8);
the group receiving HuBA-1-3D (0.3 mg/kg body weight) (N = 8);
the group receiving HuBA-1-3D (1 mg/kg body weight) (N = 8);
the group receiving CH518324/Debio1347 (50 mg/kg body weight) and HuBA-1-3D (0.3 mg/kg body weight) (N = 8); and
the group receiving CH518324/Debio1347 (50 mg/kg body weight) and HuBA-1-3D (1 mg/kg body weight) (N = 8).

HuBA-1-3D was intraperitoneally administered once a day 4 times in total on Days 14, 18, 21 and 24, while CH518324/Debio1347 was orally administered once a day 10 times in total on Days 14, 15, 16, 17, 18, 21, 22, 23, 24 and 25. The combined administration of HuBA-1-3D and CH518324/Debio1347 was performed 4 times in total on Days 14, 18, 21 and 24. In each group, a mouse whose tumor volume exceeded 1,500 mm³ was sacrificed at that point of time.

On Day 31 (i.e., at 6 days after the final day of administration (Day 25)) when all mice in all the groups did not reach the end point, the mean tumor volume in the Vehicle group (N = 8) was 1198.2 ± 285.6 mm³, whereas the mean tumor volume was 673.8 ± 271.8 mm³ in the group receiving CH518324/Debio1347 (50 mg/kg) (N = 8) (T/C = 56.2%, ^{∗}P<0.05), 1256.1 ± 465.7 mm³ in the group receiving HuBA-1-3D (0.3 mg/kg) (N = 8) (T/C = 104.8%, not significance), 66.7 ± 40.5 mm³ in the group receiving HuBA-1-3D (1 mg/kg) (N = 8) (T/C = 5.6%, ^{∗}P<0.05), 714.2 ± 296.4 mm³ in the group receiving HuBA-1-3D (0.3 mg/kg) + CH518324/Debio1347 (50 mg/kg) (N = 8) (T/C = 59.6%, ^{∗}P<0.05), and 66.7 ± 88.4 mm³ in the group receiving HuBA-1-3D (1 mg/kg) + CH518324/Debio1347 (50 mg/kg) (N = 8) (T/C = 5.6%, *P<0.01). Thus, statistically significant and potent antitumor activity was observed in the group receiving CH518324/Debio1347 (50 mg/kg), the group receiving HuBA-1-3D (1 mg/kg) and the group receiving HuBA-1-3D (0.3 or 1 mg/kg) + CH518324/Debio1347 (50 mg/kg) when compared to the Vehicle group.

On the other hand, as to the combined effect, in the group receiving HuBA-1-3D (0.3 mg/kg or 1 mg/kg) and CH518324/Debio1347 (50 mg/kg), no enhancement in the antitumor effect was observed in either combination, even when the group receiving HuBA-1-3D and CH518324/Debio1347 was compared with each of the groups receiving them alone.

The results obtained above are shown in Figure 8.

The group receiving CH518324/Debio1347 (50 mg/kg) (open squares) and the group receiving HuBA-1-3D (0.3 mg/kg) + CH518324/Debio1347 (50 mg/kg) (solid triangles) showed almost completely the same tumorigenesis curve until Day 35, while the group receiving HuBA-1-3D (1 mg/kg) (open diamonds) and the group receiving HuBA-1-3D (1 mg/kg) + CH518324/Debio1347 (50 mg/kg) (solid circles) showed almost completely the same tumorigenesis curve until Day 38 (i.e., until the final day of the test). Thus, no enhancing effect on the antitumor effect was observed upon combined administration of HuBA-1-3D antibody and CH518324/Debio1347, which is a selective tyrosine kinase inhibitor against FGFR1/FGFR2/FGFR3.

### Industrial Applicability

The present invention enables the provision of a therapeutic agent and a therapeutic method, etc., capable of exerting a more potent and sustained antitumor effect than existing tyrosine kinase inhibitors in the treatment of cancer, particularly in the treatment of cancers where FGFR4 is expressed and/or the tyrosine kinase activity of FGFR4 is observed. Thus, the therapeutic agent and therapeutic method, etc., of the present invention are very useful, for example, in term of being capable of exerting an effect on patients who could not have been expected to experience any therapeutic effect.

### Sequence Listing Free Text

SEQ ID NO: 23: recombinant DNA
SEQ ID NO: 24: synthetic construct (recombinant protein)
SEQ ID NO: 25: recombinant DNA
SEQ ID NO: 26: synthetic construct (recombinant protein)
SEQ ID NO: 27: recombinant DNA
SEQ ID NO: 28: synthetic construct (recombinant protein)
SEQ ID NO: 29: recombinant DNA
SEQ ID NO: 30: synthetic construct (recombinant protein)
SEQ ID NO: 31: recombinant DNA
SEQ ID NO: 32: synthetic construct (recombinant protein)
SEQ ID NO: 33: recombinant DNA
SEQ ID NO: 34: synthetic construct (recombinant protein)
SEQ ID NO: 35: recombinant DNA
SEQ ID NO: 36: synthetic construct (recombinant protein)
SEQ ID NO: 37: recombinant DNA
SEQ ID NO: 38: synthetic construct (recombinant protein)
SEQ ID NO: 39: recombinant DNA
SEQ ID NO: 40: synthetic construct (recombinant protein)
SEQ ID NO: 41: recombinant DNA
SEQ ID NO: 42: synthetic construct (recombinant protein)
SEQ ID NO: 43: recombinant DNA
SEQ ID NO: 44: synthetic construct (recombinant protein)
SEQ ID NO: 45: recombinant DNA
SEQ ID NO: 46: synthetic construct (recombinant protein)

| | | |
|---|---|---|
| 0-1 | Form PCT/RO/134 Indications Relating to Deposited Microorganism(s) or Other Biological Material (PCT Rule 13bls) | |
| 0-1-1 | Prepared Using | JP0-PAS |
| | | i 430 |
| 0-2 | International Application No. | |
| 0-3 | Applicant's or agent's file reference | G2716W0 |
| | | |
| 1 | The indications made below relate to the deposited microorganlsm(s) or other biological material referred to in the description on: | |
| 1-1 | **Paragraph number** | 0052 |
| 1-3 | **Identification of deposit** | |
| 1-3-1 | Name of depositary institution | IPOD International Patent Organism Depo sitary (IPOD), National Institute of Tec hnology and Evaluation (NITE) |
| 1-3-2 | Address of depositary institution | #120, 2-5-8 Kazusakamatari, Kisarazu-shi , Chiba 292-0818, Japan |
| 1-3-3 | Date of deposit | **21 August 2007 (21.08.2007)** |
| 1-3-4 | Accession Number | IPOD PERM BP-10899 |
| 1-5 | **Designated States for Which Indications are Made** | All designations |
| 2 | **The indications made below relate to the deposited microorganism(s) or other biological material referred to in the description on:** | |
| 2-1 | **Paragraph number** | 0052 |
| 2-3 | **Identification of deposit** | |
| 2-3-1 | Name of depositary institution | IPOD International Patent Organism Depo sitary (IPOD), National Institute of Tec hnology and Evaluation (NITE) |
| 2-3-2 | Address of depositary institution | #120, 2-5-8 Kazusakamatari, Kisarazu-shi , Chiba 292-0818, Japan |
| 2-3-3 | Date of deposit | **18 October 2006 (18.10.2006)** |
| 2-3-4 | Accession Number | IPOD PERM BP-10707 |
| 2-5 | **Designated States for Which Indications are Made** | All designations |
| 3 | **The Indications made below relate to the deposited microorganism(s) or other biological material referred to in the description on:** | |
| 3-1 | **Paragraph number** | 0052 |
| 3-3 | **Identification of deposit** | |
| 3-3-1 | Name of depositary institution | IPOD International Patent Organism Depo sitary (IPOD), National Institute of Tec hnology and Evaluation (NITE) |
| 3-3-2 | Address of depositary institution | #120, 2-5-8 Kazusakamatari, Kisarazu-shi , Chiba 292-0818, Japan |
| 3-3-3 | Date of deposit | 21 **August 2007 (21.08.2007)** |
| 3-3-4 | Accession Number | IPOD FERM BP-10900 |
| 3-5 | **Designated States for Which Indications are Made** | All designations |
| 4 | The Indications made below relate to the deposited microorganism(s) or other biological material referred to In the description on: | |
| 4-1 | **Paragraph number** | 0052 |
| 4-3 | **Identification of deposit** | |
| 4-3-1 | Name of depositary institution | IPOD International Patent Organism Depo sitary (IPOD), National Institute of Tec hnology and Evaluation (NITE) |
| 4-3-2 | Address of depositary institution | #120, 2-5-8 Kazusakamatari, Kisarazu-shi , Chiba 292-0818, Japan |
| 4-3-3 | Date of deposit | **01 February 2011 (01.02.2011)** |
| 4-3-4 | Accession Number | IPOD PERM BP-11337 |
| 4-5 | **Designated States for Which Indications are Made** | All designations |

### FOR RECEIVING OFFICE USE ONLY

| | | |
|---|---|---|
| 04 | **This form was received with the International application:** (yes or no) | |
| 0-4-1 | Authorized officer | |

### FOR INTERNATIONAL BUREAU USE ONLY

| | | |
|---|---|---|
| 0-5 | **This form was received by the international Bureau on:** | |
| 0-5-1 | Authorized officer | |

## Claims

1. A pharmaceutical combination for the treatment of cancer, comprising:
a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
an antibody against human DLK-1, which has *in vivo* antitumor activity, or an antibody fragment derived from the antibody.

2. A pharmaceutical composition for the treatment of cancer, comprising an antibody against human DLK-1, which has *in vivo* antitumor activity, or an antibody fragment derived from the antibody,
wherein the pharmaceutical composition is used in combination with a substance which inhibits or suppresses the tyrosine kinase activity of fibroblast growth factor receptor 4 (FGFR4), or a prodrug thereof, or a pharmacologically acceptable salt thereof, or a hydrate or solvate thereof.

3. A pharmaceutical composition for the treatment of cancer, comprising a substance which inhibits or suppresses the tyrosine kinase activity of fibroblast growth factor receptor 4 (FGFR4), or a prodrug thereof, or a pharmacologically acceptable salt thereof, or a hydrate or solvate thereof,
wherein the pharmaceutical composition is used in combination with an antibody against human DLK-1, which has *in vivo* antitumor activity, or an antibody fragment derived from the antibody.

4. A pharmaceutical composition for the treatment of cancer, comprising an antibody against human DLK-1, which has *in vivo* antitumor activity, or an antibody fragment derived from the antibody,
wherein the treatment comprises administering a substance which inhibits or suppresses the tyrosine kinase activity of fibroblast growth factor receptor 4 (FGFR4), or a prodrug thereof, or a pharmacologically acceptable salt thereof, or a hydrate or solvate thereof.

5. A pharmaceutical composition for the treatment of cancer, comprising a substance which inhibits or suppresses the tyrosine kinase activity of fibroblast growth factor receptor 4 (FGFR4), or a prodrug thereof, or a pharmacologically acceptable salt thereof, or a hydrate or solvate thereof,
wherein the treatment comprises administering an antibody against human DLK-1, which has *in vivo* antitumor activity, or an antibody fragment derived from the antibody.

6. The pharmaceutical combination according to claim 1, or the pharmaceutical composition according to any one of claims 2 to 5, wherein the substance which inhibits or suppresses the tyrosine kinase activity of fibroblast growth factor receptor 4 (FGFR4) is H3B-6527, FGF-401 (roblitinib) or BLU-554 (fisogatinib).

7. The pharmaceutical combination according to claim 1, or the pharmaceutical composition according to any one of claims 2 to 5, wherein the cancer and/or the tumor is a cancer where FGFR4 is expressed and/or the tyrosine kinase activity of FGFR4 is observed.

8. The pharmaceutical combination according to claim 1, or the pharmaceutical composition according to any one of claims 2 to 5, wherein the cancer and/or the tumor is at least one selected from the group consisting of hepatocellular carcinoma, lung cancer, uterine body cancer, cholangiocarcinoma, intrahepatic bile duct cancer, esophageal cancer, nasopharyngeal cancer, ovarian cancer, breast cancer, renal cell carcinoma, pancreatic cancer, colon cancer, and glioblastoma, as well as sarcomas including rhabdomyosarcoma.

9. The pharmaceutical combination according to claim 1, or the pharmaceutical composition according to any one of claims 2 to 5, wherein the antibody is a chimeric antibody or a humanized antibody.

10. The pharmaceutical combination according to claim 1, or the pharmaceutical composition according to any one of claims 2 to 5, wherein the antibody is at least one selected from the group consisting of:
(a) an antibody in which the amino acid sequences of H chain V region CDRs 1 to 3 are the amino acid sequences shown in SEQ ID NOs: 3 to 5, respectively, and the amino acid sequences of L chain V region CDRs 1 to 3 are the amino acid sequences shown in SEQ ID NOs: 6 to 8, respectively;
(b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
(c) an antibody in which the amino acid sequence of the H chain V region consists of the amino acid sequence shown in SEQ ID NO: 16, and the amino acid sequence of the L chain V region consists of the amino acid sequence shown in SEQ ID NO: 18;
(d) an antibody in which the amino acid sequence of the H chain V region consists of the amino acid sequence shown in SEQ ID NO: 20, and the amino acid sequence of the L chain V region consists of the amino acid sequence shown in SEQ ID NO: 22;
(e) an antibody in which the amino acid sequence of the H chain V region consists of the amino acid sequence shown in SEQ ID NO: 24 or 26, and the amino acid sequence of the L chain V region consists of the amino acid sequence shown in SEQ ID NO: 28;
(f) an antibody in which the amino acid sequence of the H chain V region consists of the amino acid sequence shown in SEQ ID NO: 30, 32, 34 or 36, and the amino acid sequence of the L chain V region consists of the amino acid sequence shown in SEQ ID NO: 46;
(g) an antibody in which the amino acid sequence of the H chain V region consists of the amino acid sequence shown in SEQ ID NO: 38, 40, 42 or 44, and the amino acid sequence of the L chain V region consists of the amino acid sequence shown in SEQ ID NO: 46;
(h) an antibody produced by the hybridoma of Accession No. FERM BP-10899;
(i) an antibody produced by the hybridoma of Accession No. FERM BP-10707;
(j) an antibody produced by the hybridoma of Accession No. FERM BP-10900; and
(k) an antibody produced by the hybridoma of Accession No. FERM BP-11337.

11. The pharmaceutical combination according to claim 1, or the pharmaceutical composition according to any one of claims 2 to 5, wherein the antibody or antibody fragment is in the form of a conjugate with a compound having antitumor activity and/or cell killing activity.

12. The pharmaceutical combination according to claim 1, or the pharmaceutical composition according to any one of claims 2 to 5, which allows suppression of cancer cell proliferation or allows tumor reduction or disappearance even after completing the administration of the pharmaceutical combination or pharmaceutical composition.

13. The use of:
a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
an antibody against human DLK-1, which has *in vivo* antitumor activity, or an antibody fragment derived from the antibody for the manufacture of a therapeutic agent for cancer.

14. A therapeutic method for cancer, **characterized by** administering a subject with:
a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
an antibody against human DLK-1, which has *in vivo* antitumor activity, or an antibody fragment derived from the antibody.

15. A kit for the treatment of cancer, comprising:
a substance which inhibits or suppresses the tyrosine kinase activity of fibroblast growth factor receptor 4 (FGFR4), or a prodrug thereof, or a pharmacologically acceptable salt thereof, or a hydrate or solvate thereof; and
an antibody against human DLK-1, which has *in vivo* antitumor activity, or an antibody fragment derived from the antibody.

16. The use according to claim 13, the method according to claim 14, or the kit according to claim 15, wherein the cancer and/or the tumor is a cancer where FGFR4 is expressed and/or the tyrosine kinase activity of FGFR4 is observed.
